(19) Europäisches Patentamt
European Patent Office
Office européen des brevets

(11) EP 1 022 058 A1

(12) **EUROPEAN PATENT APPLICATION**
published in accordance with Art. 158(3) EPC

(43) Date of publication:
**26.07.2000 Bulletin 2000/30**

(21) Application number: **99919529.0**

(22) Date of filing: **12.05.1999**

(51) Int. Cl.[7]: **B01J 31/06**

(86) International application number:
**PCT/JP99/02438**

(87) International publication number:
**WO 99/58242 (18.11.1999 Gazette 1999/46)**

(84) Designated Contracting States:
**DE FR GB**

(30) Priority: **12.05.1998 JP 12908098**
**11.05.1999 JP 12980499**

(71) Applicant:
**Nippon Shokubai Co., Ltd.**
**Osaka-shi, Osaka 541-0043 (JP)**

(72) Inventors:
- **TAKAHASHI, Yoshiyuki**
  **Kyoto-shi Kyoto 601-8206 (JP)**
- **YAMAMOTO, Hiroshi**
  **Suita-shi Osaka 564-0024 (JP)**
- **HIRANO, Yoshiaki**
  **Nishinomiya-shi Hyogo 663-8004 (JP)**
- **MORITA, Takehiko**
  **Ibaraki-shi Osaka 567-0023 (JP)**
- **KUBO, Takafumi**
  **Suita-shi Osaka 564-0035 (JP)**
- **OMOTO, Ichiro**
  **Himeji-shi Hyogo 671-1205 (JP)**

(74) Representative:
**West, Alan Harry et al**
**R.G.C. Jenkins & Co.**
**26 Caxton Street**
**London SW1H 0RJ (GB)**

# (54) METHOD FOR ACTIVATING ACTIVE HYDROGEN AND ACTIVATION CATALYST

(57) The ESPD charge of at least one atom in an atomic group forming a functional group included in an activating catalyst is in a range between -0.65 and -1.05 (electrons). Alternately, an activating catalyst includes a cyclic quaternary ammonium group as the functional group and the ESPD charge of a nitrogen atom forming the ammonium group is in a range between +0.4 and +1.3 (electrons). Consequently, the catalyst can efficiently activate an active hydrogen included in a compound using the atom showing the ESPD charge in the above-specified range as an active site. In other words, the catalyst can be suitably used as an activating catalyst for various reactions involving the activation of the active hydrogen. Also, by using the activating catalyst, a reaction with excellent selectivity can be conducted.

EP 1 022 058 A1

## Description

TECHNICAL FIELD

The present invention relates to a method of activating an active hydrogen included in a compound, and also to an activating catalyst which activates the active hydrogen.

TECHNICAL BACKGROUND

Studies have been carried out for many reaction processes using a catalyst in, for example, a hydration reaction or a polymerization reaction of olefin. The catalyst includes: (1) a homogeneous catalyst, represented by an acid catalyst used in a solution and having the catalyst phase and reaction phase within a single phase; and (2) a heterogeneous catalyst, represented by a solid catalyst added to a reaction system in a gaseous or liquid phase and having the catalyst phase and reaction phase separately.

In case of the homogenous catalyst, active ingredients, which activate an active hydrogen included in a compound used as a reaction substrate, reside in the phase where the reaction phase also resides. Therefore, for example, the reaction proceeds with the active ingredients being diffused homogeneously in the solution. Thus, when the homogeneous catalyst is used, a reaction rate and a conversion ratio of the reaction substrate are both high. Hence, the catalyst can be designed easily when the homogeneous catalyst is used. On the other hand, the homogeneous catalyst can not be separated readily and a waste solution containing the catalyst is produced after the reaction, thereby posing a problem that the waste solution has to be treated.

On the contrary, in case of the heterogeneous catalyst, the active ingredients and reaction phase reside separately. Therefore, the catalyst can be readily separated, and no waste solution containing the catalyst is produced after the reaction. Thus, the heterogeneous catalyst is advantageous over the homogeneous catalyst in terms of the post-reaction treatment.

However, because the heterogenous catalyst undergoes the reaction using a functional group fixed to a carrier or the surface of a host as an active site like ion-exchange resin, for example, the reaction takes place on the surface of the catalyst. For this reason, when the heterogeneous catalyst is used, a reaction rate and a conversion ratio of the reaction substrate are both low. Hence, when the heterogeneous catalyst is used, there is a problem that the catalyst can not be designed easily.

Under these circumstances, there has been a need for a method of facilitating the catalyst design so as to develop a heterogeneous catalyst having a high reaction rate and a high conversion ratio of the reaction substrate, for example. Accordingly, there also has been a need for a method of readily evaluating a catalytic ability of the designed catalyst. Further, there has been a need for an activating catalyst suitably used as a catalyst in various reactions involving the activation of the active hydrogen included in a compound used as the reaction substrate, and a method of activating the active hydrogen included in the compound.

The present invention is devised to solve the above problems, and therefore, has an object to provide a method of activating an active hydrogen included in a compound, and a catalyst which activates the active hydrogen, that is, an activating catalyst used suitably as a catalyst in various reactions involving the activation of the active hydrogen included in the compound. The present invention has another object to provide a method of readily evaluating the catalytic ability of the catalyst.

DISCLOSURE OF THE INVENTION

In order to achieve the above and other objects, the inventors of the present invention conducted an assiduous study on the method of activating the active hydrogen and the activating catalyst. In due course, the inventors discovered that the catalytic ability of the catalyst can be readily evaluated by an ESPD (Electrostatic Potential Derived) charge. The inventors also discovered that if the ESPD charge of at least one atom in an atomic group forming a functional group is in a specific range, such a catalyst can activate the active hydrogen included in the compound efficiently by using the atom showing the ESPD charge in the specified range as an active site. In other words, the inventors discovered that such a catalyst is suitable as an activating catalyst in various reactions involving the activation of the active hydrogen. The inventors also discovered that if the catalyst includes a cyclic quaternary ammonium group as the functional group and the ESPD charge of a nitrogen atom forming the ammonium group is in a specific range, such a catalyst can efficiently activate the active hydrogen included in the compound using the nitrogen atom as the active site. In other words, the inventors discovered that such a catalyst is suitable as an activating catalyst in various reactions involving the activation of the active hydrogen. Finally, the inventors achieved the present invention when they discovered that the catalytic ability of the catalyst can be readily evaluated by the ESPD charge.

Documents (Journal of Molecular Structure (Theochem), 201 (1989) 257-270) have been known as describing a correlation between the ESP (Electrostatic Potential) and a basic property (pKa) in each kind of compound. However, the above documents fail to describe the relation between the ESPD charge and basic property or how the largeness of the ESPD charge affects the reactivity.

In order to fulfill the above and other objects, an activating catalyst for the active hydrogen of the present invention is a catalyst which activates the active hydrogen included in a compound, and characterized in that the ESPD charge of at least one atom in an atomic group forming the functional group included in the catalyst is in a range between -0.65 and -1.05 (electrons). Also, the activating catalyst for the active hydrogen of the present invention is characterized in that the atom showing the ESPD charge in the above-specified range is at least one kind of atom selected from the group consisting of a nitrogen atom, an oxygen atom, and a sulfur atom. Further, the activating catalyst for the active hydrogen of the present invention is characterized in that the functional group which includes the atom showing the ESPD charge in the above-specific range is a cyclic amino group.

Also, in order to fulfill the above and other objects, the activating catalyst for the active hydrogen of the present invention is a catalyst which activates the active hydrogen included in a compound, and characterized in that it has a cyclic quaternary ammonium group as the functional group, and the ESPD charge of a nitrogen atom forming the ammonium group is in a range between +0.4 and +1.3 (electrons). Further, the activating catalyst for the active hydrogen of the present invention is characterized in that the cyclic quaternary ammonium group has 5 or 6 members.

In order to fulfill the above and other objects, the method of activating the active hydrogen of the present invention is characterized by activating the active hydrogen included in a compound using the above-arranged activating catalyst.

Also, in order to fulfill the above and other objects, the method of evaluating the activating catalyst of the present invention is characterized in that the catalytic ability of the catalyst to activate the active hydrogen included in a compound is evaluated by the ESPD charge of an atom forming the functional group of the catalyst.

Further objects, the nature and advantages of the invention will be understood by the following description. Also, the effects of the present invention will be explained clearly in the following description.

## THE MOST PREFERRED EMBODIMENT OF THE INVENTION

The ESPD (Electrostatic Potential Derived) charge referred to herein means a value computed by a semi-experimental MO (Molecular Orbital) method computation using a comprehensive molecular design aid system, commercially known as Spartan® (version 5.0) of Wavefunction, Inc. Although Spartan® (version 5.0) is packed with a document (Experiments in Computational Organic Chemistry) describing the relation between the ESPD charge and acidic properties as an appendix, the document does not describe the relation between the ESPD charge and basic properties, nor how a largeness of the ESPD charge affects the reactivity.

The activating catalyst for the active hydrogen of the present invention is a catalyst which activates an active hydrogen included in a compound, and the ESPD charge of at least one atom in an atomic group forming a functional group of the catalyst is preferably in a range between -0.65 and -1.05 (electrons), more preferably in a range between -0.65 and -1.00, and most preferably in a range between -0.70 and -0.95. Also, the activating catalyst for the active hydrogen of the present invention is a catalyst which activates the active hydrogen included in a compound and has a cyclic quaternary ammonium group as the functional group, while the ESPD charge of a nitrogen atom forming the ammonium group is preferably in a range between +0.4 and +1.3 (electrons), more preferably in a range between +0.50 and +1.2, and most preferably in a range between +0.55 and +1.1. In the following explanation, when the above activating catalysts need to be distinguished from each other, the former is referred to as the activating catalyst ①and the latter the activating catalyst ②.

The method of activating the active hydrogen of the present invention is a method of activating the active hydrogen included in a compound using the above-arranged activating catalyst. Further, the method of evaluating the activating catalyst of the present invention is a method of evaluating the catalytic ability of the catalyst to activate the active hydrogen included in a compound by the ESPD charge of an atom forming the functional group included in the catalyst (in case of the activating catalyst ②, the atom includes a nitrogen atom forming the cyclic quaternary ammonium group).

The structure of the activating catalyst ①is not especially limited as long as the ESPD charge of at least one atom in the atomic group forming the functional group included in the catalyst is in the range specified above. The structure of the activating catalyst ②is not especially limited, either, as long as it has a cyclic quaternary ammonium group as the functional group and the ESPD charge of a nitrogen atom forming the ammonium group is in the range specified above.

However, the activating catalyst is preferably a crosslinking macromolecule of a 3-D net structure from the view points of catalytic ability and strength. The activating catalyst more preferably has an active site on the surface and/or in the internal of the structure, at which the active hydrogen included in a compound is activated. By the above arrangement, the active hydrogen included in the compound used as a reaction substrate (hereinafter, referred to as

the active-hydrogen-containing compound) can be activated more efficiently. Thus, both the reaction rate and conversion ratio of the active-hydrogen-containing compound can be improved. In addition, the crosslinking macromolecule can be readily separated and removed from the reaction system after the reaction. The following will describe a case where the activating catalyst is a crosslinking macromolecule. It is preferable that the crosslinking macromolecule does not react or hardly reacts with the active-hydrogen-containing compound and a solvent.

The crosslinking macromolecule which will be made into the activating catalyst ①(hereinafter, referred to as the crosslinking macromolecule ①when it needs to be distinguished from the crosslinking macromolecule which will be made into the activating catalyst ②) preferably uses a monomer having at least one of an acidic functional group and a basic functional group as a polymerization unit. Therefore, examples of the acidic functional group of the crosslinking macromolecule ①include, but not limited to: a carboxyl group, a thiocarboxyl group, a sulfonic acid group, a phosphoric acid group, a phosphorous acid group, a hydroxyl group, a phenolic hydroxyl group, a thiol group, a thiophenolic thiol group, etc. Of all these examples of the acidic functional group, a carboxyl group, a sulfonic acid group, a phosphoric acid group, a phosphorous acid group, a hydroxyl group are more preferable than the others. The crosslinking macromolecule ①may include more than one kind of the acidic functional group. Here, the acidic functional group includes esters of an acid, in which a hydrocarbon group substitutes a hydrogen atom of the acidic functional group, and salts of an acid, in which a metal ion substitutes a hydrogen atom.

Examples of the basic functional group included in the crosslinking macromolecule ①include, but not limited to:

functional groups, such as a cyano group, an isocyano group, a thiocyano group, and an isothiocyano group; functional groups derived from a basic compound, such as amines, amides, thioamides, a heterocyclic compound (for example, pyrroles, thiophenes, imidazoles, pyrrolidines, piperidines, piperazines, oxazoles, pyridines, pyrimidines, pyrazines, pyridazines, purines, thiazoles, pyrazoles, carbazoles, etc.), represented by a quaternary salt, in which one hydrogen ion is added to amines of the above-specified basic compound; etc. Of all these examples of the basic functional group, the functional groups derived from the basic compound, such as amines, thiophenes, pyridines, and carbazoles, are more preferable than the others. The crosslinking macromolecule ①may include more than one kind of the basic functional group.

Because the crosslinking macromolecule ①has at least one of the acidic functional group and basic functional group, it can exhibit a catalytic ability with excellent selectivity. Also, in case that the crosslinking macromolecule ①has both the acidic functional group and basic functional group, it has both the acidic site and basic site. Therefore, these sites function in cooperation, and the crosslinking macromolecule ①can exhibit a catalytic ability with more excellent selectivity.

The crosslinking macromolecule ①can be readily obtained by polymerizing a monomer component which contains a monomer having at least one of the acidic functional group and basic functional group. Besides the monomer having the functional group, the monomer component may contain a copolymerizible monomer capable of copolymerizing with such a monomer. Examples of the monomer having the acidic functional group include, but not limited to:

a monomer expressed by General Formula (1):

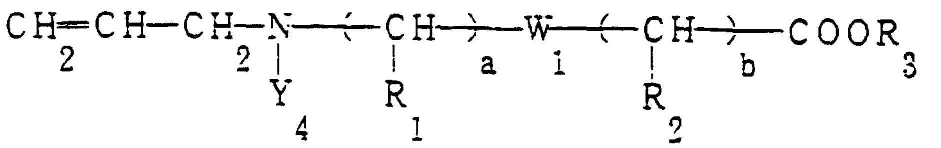

...... (1)

where $Y_4$ represents a hydrogen atom, an allyl group, or a - $(-CHR_4-)_c-W_2-(-CHR_5-)_d-COOR_6$ group, each of $R_1$, $R_2$, $R_3$, $R_4$, $R_5$, and $R_6$ independently represents a hydrogen atom, or an alkyl group having 1-6 carbon atoms, each of $W_1$ and $W_2$ independently represents a -NH- group, a -S- group, or a -O- group, and each of a, b, c, and d independently represents an integer from 0 to 6, and

a carboxylic acid monomer, such as (meth)acrylic acid, a maleic acid, and a fumaric acid; etc. One member or a mixture of two or more members selected from these examples can be effectively used as the above monomer.

Examples of the monomer having the basic functional group include, but not limited to:

vinyl pyridines, such as 2-vinyl pyridine and 4-vinyl pyridine;

allylamines, such as N-vinyl carbazoles, N-monoallylamines, and N,N-diallylamines, N,N,N-triallylamines;
4-(N,N-dialkylamino)alkylstyrenes;
6-(N-propenylamino)-4-thiahexanoic acid;
6-(N,N-dipropenylamino)-4-thiahexanoic acid; etc.

One member or a mixture of two or more members selected from these examples can be used effectively as the above monomer.

The copolymerizible monomer is not especially limited as long as it is a monomer including an olefin group and excluding an acidic functional group and a basic functional group. Examples of the copolymerizible monomer include styrene, ethylene, vinyl ethers, etc. One member or a mixture of two or more members selected from these examples can be used effectively as the copolymerizible monomer, as the case may be. Here, a ratio of a used amount of the copolymerizible monomer with respect to that of the monomer having the acidic functional group or basic functional group is not especially limited.

A method of manufacturing the crosslinking macromolecule ①, that is, a method of polymerizing the monomer component, is not especially limited. For example, various known methods, such as solution polymerization, suspension polymerization, and reverse phase suspension polymerization, can be adopted. Examples of a solvent used in polymerizing the monomer component include, but not limited to: water, toluene, cyclohexane, etc. Further, examples of a suspension agent used in the suspension polymerization include, but not limited to: ethyl cellulose, gelatin, dextrin, polyvinyl alcohol, sorbitan esters, etc. Used amounts of the solvent and suspension agent are not especially limited, either.

A polymerization initiator can be used when polymerizing the monomer component. A radical polymerization initiator can be used as the polymerization initiator, examples of which include:

peroxides, such as hydrogen peroxide, benzoyl peroxide, and cumene hydroperoxide;
azo compounds, such as 2,2'-azobisisobutylonitrile and 2,2'-azobis-(2-amidinopropane)dihydrochloride;
persulfates, such as ammonium persulfate, sodium persulfate, and potassium persulfate; etc. One member or a mixture of two or more members selected from these examples can be used effectively as the polymerization initiator. Alternatively, activation energy rays, electron beams, UV rays, etc. can be used instead of, or together with the polymerization initiator. Here, a used amount of the polymerization initiator is not especially limited.

A reaction temperature for the above polymerization reaction is not especially limited, and can be set arbitrary depending on the kinds and a combination of the monomer component, solvent, and polymerization initiator, and the other factors. Also, a reaction time can be set adequately so as to complete the above polymerization reaction depending on the reaction temperature, the kinds, a combination, and used amounts of the monomer component, solvent, and polymerization initiator and the other factors. Further, a reaction pressure is not especially limited, and the polymerization reaction can be conducted under any of a normal (atmospheric) pressure, a reduced pressure, or an applied pressure.

By using a crosslinking monomer (crosslinking agent) for the above polymerization reaction, a degree of crosslinking of the resulting crosslinking macromolecule ①can be controlled. In other words, the monomer component may further include the crosslinking monomer. Examples of the crosslinking monomer include, but not limited to: trimethylol propane triacrylate, polyethylene glycol diacrylate, methylenebis acrylamide, divinyl benzene, etc. Alternatively, a compound containing an epoxy group, such as epichlorohydrin, can be used for the crosslinking.

A degree of crosslinking of the crosslinking macromolecule ①is indicated by a molar fraction of the crosslinking monomer in the monomer component, and generally in a range between 0.1 and 30 mol%, preferably in a range between 0.1 and 10 mol%, and more preferably in a range between 0.1 and 5 mol%.

In addition, the following method can be adopted as a method of manufacturing the crosslinking macromolecule ①. That is, after a polymer having primary or secondary alkyl halogen or a sulfonic acid ester as a side chain is reacted with any kind of sulfurizing agent, a -SH group is introduced to an end terminal of the polymer by reducing the resulting intermediate by means of hydrolysis, a treating agent, etc. Then, ethylenic unsaturated carboxylic acid, such as acrylic acid and methacrylic acid, is added to the polymer.

Examples of the crosslinking macromolecule ①obtained from the above polymerization reaction include,

a crosslinking macromolecule having a functional group of a structure expressed by General Formula (2):

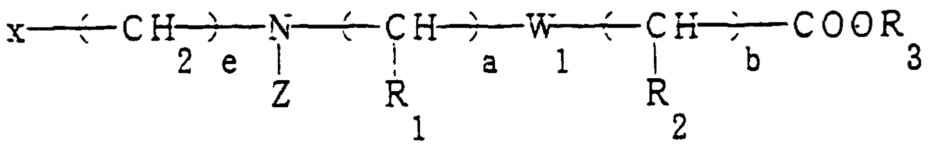 ...... (2)

where Z represents a hydrogen atom, a - $(-CHR_4-)_c-W_2-(-CHR_5-)_d-COOR_6$ group, or a $-(-CH_2-)_f-x$ group, each of $R_1$, $R_2$, $R_3$, $R_4$, $R_5$, and $R_6$ independently represents a hydrogen atom, or an alkyl group having 1-6 carbon atoms, each of $W_1$ and $W_2$ independently represents a -NH-group, a -S-group, or a -O- group, x represents a carboxylic acid polymer main body, and each of a, b, c, d, e, and f independently represents an integer from 0 to 6;

a crosslinking macromolecule having a functional group of a structure expressed by General Formula (3):

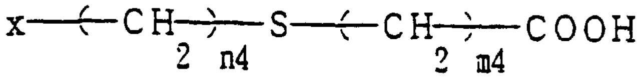 ...... (3)

where x represents a carboxylic acid polymer main body, $n_4$ represents an integer from 0 to 6, and $m_4$ represents an integer from 0 to 6;

a crosslinking macromolecule having a functional group of a structure expressed by at least one general formula selected from the group consisting of General Formulas (4) through (8):

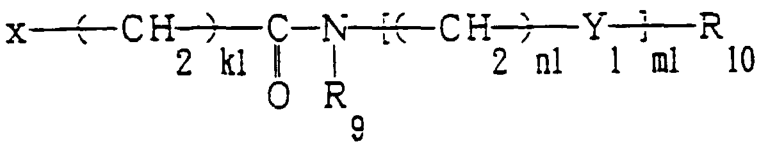 ...... (4)

where each of $R_9$ and $R_{10}$ independently represents a hydrogen atom, a hydrocarbon group having 1-5 carbon atoms, or a $-(-CH_2-)_{p1}-x$ group, $p_1$ represents an integer from 0 to 6, $k_1$ represents an integer from 0 to 6, $m_1$ represents an integer from 0 to 6, $n_1$ represents an integer from 1 to 6, $Y_1$ represents a -O- group, a -S-group, or a -$NR_{11}$- group, $R_{11}$ represents a hydrogen atom or a hydrocarbon having 1-5 carbon atoms, and x represents a carboxylic acid polymer main body;

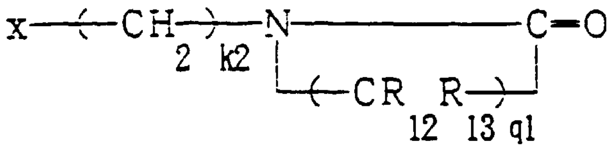 ...... (5)

where each of $R_{12}$ and $R_{13}$ independently represents a hydrogen atom or a hydrocarbon group having 1-5 carbon atoms, $K_2$ represents an integer from 0 to 6, $q_1$ represents an integer from 3 to 6, and x represents a carboxylic acid polymer main body;

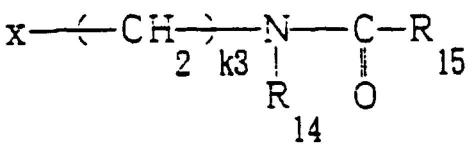 ...... (6)

where $R_{14}$ represents a hydrogen atom, a hydrocarbon group having 1-5 carbon atoms, or a $-(-CH_2-)_{p2}-x$ group, $p_2$ represents an integer from 0 to 6, $R_{15}$ represents a hydrocarbon group having 1-5 carbon atoms, or a $-(-CH_2-)_{p3}-x$ group, $p_3$ represents an integer from 0 to 6, $k_3$ represents an integer from 0 to 6, and x represents a carboxylic acid polymer main body;

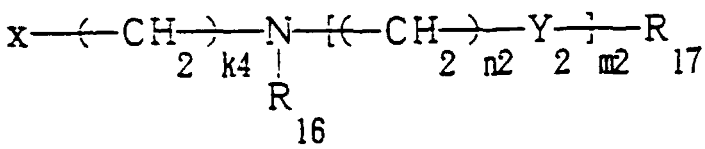
...... (7)

where $R_{16}$ represents a hydrogen atom, a hydrocarbon group having 1-5 carbon atoms, or a $-(-CH_2-)_{p4}-x$ group, $p_4$ represents an integer from 0 to 6, $k_4$ represents an integer from 0 to 6, $n_2$ represents an integer from 1 to 6, $Y_2$ represents a -O- group, a -S- group, a $-NR_{18}-$ group, or a $-CH_2-$ group, $R_{18}$ represents a hydrogen atom or a hydrocarbon group having 1-5 carbon atoms, $m_2$ represents an integer from 0 to 6, $R_{17}$ represents a hydrogen atom, a hydrocarbon group having 1-5 carbon atoms, a $-(-CH_2-)_{p5}-x$ group, or a Brønsted acid residue when $m_2 \neq 0$, and a hydrogen atom, a hydrocarbon group having 1-5 carbon atoms, or $-(-CH_2-)_{p5}-x$ group, when $m_2=0$, $p_5$ represents an integer from 0 to 6, and x represents a carboxylic acid polymer main body;

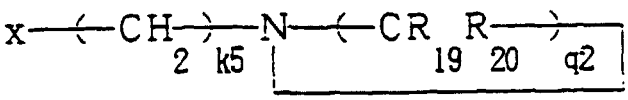
...... (8)

where each of $R_{19}$ and $R_{20}$ independently represents a hydrogen atom or a hydrocarbon group having 1-5 carbon atoms, $k_5$ represents an integer from 0 to 6, $q_2$ represents an integer from 4 to 7, and x represents a carboxylic acid polymer main body; etc.

However, the crosslinking macromolecule ①is not especially limited as long as it has a 3-D net structure insoluble to the solvent. The kinds of the functional group included in the crosslinking macromolecule ①are not especially limited, either.

In General Formulas (2) through (8) above, the carboxylic acid polymer main body means a main chain of the carboxylic acid polymer, which can be obtained either by polymerizing a monomer containing a carboxyl group alone (for example, the aforementioned carboxylic acid monomer), or copolymerizing a monomer containing a carboxyl group with a monomer copolymerizible with such a monomer.

The crosslinking macromolecule which will be made into the activating catalyst ② (hereinafter, referred to as the crosslinking macromolecule ② when it needs to be distinguished from the crosslinking macromolecule ①) preferably uses, as a polymerization unit, a monomer having a monovalent group of a cyclic amine structure capable of forming a cyclic quaternary ammonium group as a functional group. Therefore, examples of the cyclic quaternary ammonium group include, but not limited to, an ammonium group obtained by converting a monovalent group having a nitrogen-containing heterocyclic structure, such as a pyrrolidine ring composed of 5 members and a piperidine ring composed of 6 members, to a quaternary compound. The nitrogen-containing heterocyclic structure may additionally include a substituent, such as a hydrocarbon group. In addition, the crosslinking macromolecule ② includes more than one kind of the cyclic quaternary ammonium group, or another kind of functional group in addition to the cyclic quaternary ammonium group. In other words, the monomer used as the polymerization unit may have more than one kind of monovalent group each having a different cyclic amine structure, or another kind of functional group in addition to the monovalent group.

Because the crosslinking macromolecule ② includes the cyclic quaternary ammonium group as the functional group, it can exhibit a catalytic ability with excellent selectivity.

The crosslinking macromolecule ② can be readily obtained by polymerizing a monomer component containing a monomer having a monovalent group of a cyclic amine structure as the functional group, and then subjecting the resulting polymer to a quaternary reaction using a quaternary agent. Alternatively, the crosslinking macromolecule ②can be readily obtained by polymerizing a monomer component containing a divinyl compound having a quaternary ammonium salt which will be made into a cyclic quaternary ammonium group after the polymerization, and/or a diallyl compound or a triallyl compound of an amine structure which will be made into a cyclic quaternary ammonium group after the polymerization. Besides the monomer having the functional group, the monomer component may further

include a copolymerizible monomer capable of copolymerizing the above monomer. Examples of the monomer having a monovalent group of the cyclic amine structure include, but not limited to, a monomer expressed by General Formula (9):

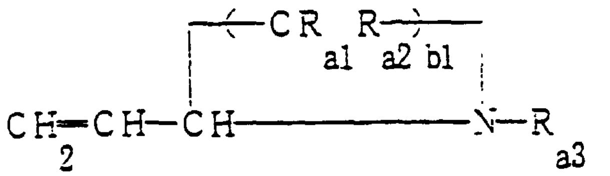
...... (9)

where each of $R_{a1}$ and $R_{a2}$ independently represents a hydrogen atom or a hydrocarbon group having 1-5 carbon atoms, $R_{a3}$ represents a hydrocarbon group having 1-5 carbon atoms, or a $-CH_2-CH=CH_2$ group, and $b_1$ represents an integer 3 or 4. Examples of the above monomer include, but not limited to:

vinylpyrrolidines, such as N-methyl-3-vinylpyrrolidine and N-(2-propenyl)-3-vinylpyrrolidine;
vinylpiperidines, such as N-methyl-3-vinylpiperidine and N-(2-propenyl)-3-vinylpiperidine; etc. Examples of a divinyl compound and a diallyl compound include, but not limited to, a monomer expressed by General Formula (10): where R' represents a hydrogen atom or a hydrocarbon group having 1-5 carbon atoms; and
a monomer expressed by General Formula (11):

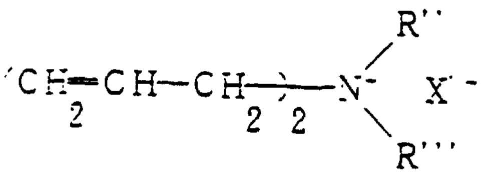
...... (11)

where each of R'' and R''' independently represents a hydrogen atom or a hydrocarbon group having 1-5 carbon atoms, $X'^-$ represents a paired anion, such as a halide ion, an $OH^-$ ion, a $CH_2=CHCOO^-$ ion, a $HCO_3^-$ ion, a carboxylic acid ion, a molybdic acid ion, a tungstic acid ion, a metavanadic acid ion, a pyrovanadic acid ion, a hydrogen pyrovanadic acid ion, and a hydrogen sulfite ion. Examples of the triallyl compound include, but not limited to: a monomer having an allyl group as the substituent R' in General Formula (2) above, and a monomer having an allyl group as the substituent R'' or R''' in General Formula (3) above. One member or a mixture of two or more members selected from these examples can be used effectively as the above monomer.

The copolymerizible monomer is not especially limited as long as it is a monomer having an olefin group. Examples of the copolymerizible monomer include styrene, ethylene, vinyl ether, etc. One member or a mixture of two or more members selected from these examples can be used effectively as the copolymerizible monomer, as the case may be. A ratio of a used amount of the copolymerizible monomer with respect to that of the monovalent group or a quaternary ammonium salt of the cyclic amine structure, or the monomer of an amine structure is not especially limited.

A method of manufacturing the crosslinking macromolecule ②, that is, a method of polymerizing the monomer component, and a method of converting the resulting crosslinked polymer to a quaternary compound are not especially limited.

For example, known methods, such as solution polymerization, suspension polymerization, reverse phase suspension polymerization, etc. can be adopted as the method of polymerizing the monomer component. A solvent used in polymerizing the monomer component is not especially limited, and can be any of the foregoing examples of the solvent. Further, the suspension agent used in the suspension polymerization is not especially limited, and can be any of the foregoing examples of the suspension agent. Here, used amounts of the solvent and suspension agent are not especially limited, either.

When the monomer component is polymerized, any of the foregoing examples of the polymerization initiator can be used. Alternatively, activation energy rays, electron beams, UV rays, etc. may be used instead of, or together with the polymerization initiator. A used amount of the polymerization initiator is not especially limited.

A reaction temperature at which the above polymerization reaction is conducted is not especially limited,

and can be set adequately depending on the kinds and a combination of the monomer component, solvent, and polymerization initiator, and other factors. Also, a reaction time can be set adequately so as to complete the polymerization reaction depending on the reaction temperature, the kinds, a combination, and used amounts of the monomer component, solvent, and polymerization initiator, and the other factors. Further, a reaction pressure is not especially limited, and the reaction can be conducted under any of a normal (atmospheric) pressure, a reduced pressure, or an applied pressure.

By using a crosslinking monomer (crosslinking agent) for the polymerization reaction, a degree of crosslinking of the resulting crosslinked polymer (crosslinking macromolecule ②) can be controlled. In other words, the monomer component can further include the foregoing cross linking monomer.

A degree of crosslinking of the crosslinked polymer (crosslinking macromolecule ②) is indicated as a molar fraction of the crosslinking monomer in the monomer component in case that the crosslinking monomer is used, and generally in a range between 0.1 and 30 mol%, preferably in a range between 0.1 and 10 mol%, and more preferably in a range between 0.1 and 5 mol%.

Examples of the crosslinked polymer obtained from the above polymerization reaction include a crosslinked polymer having a functional group of a structure expressed by General Formula (12):

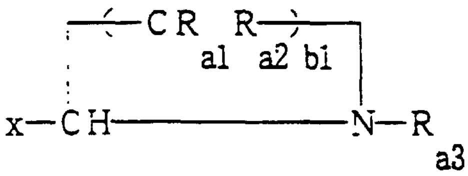

······ (12)

where each of $R_{a1}$ and $R_{a2}$ independently represents a hydrogen atom or a hydrocarbon group having 1-5 carbon atoms, $R_{a3}$ represents a hydrocarbon group having 1-5 carbon atoms or a $-CH_2-CH=CH_2$ group, b1 represents an integer of 3 or 4, and x represents a crosslinked polymer main body;

or a crosslinked polymer (in this case, the crosslinking macromolecule ②) having a functional group of a structure expressed by General Formula (13):

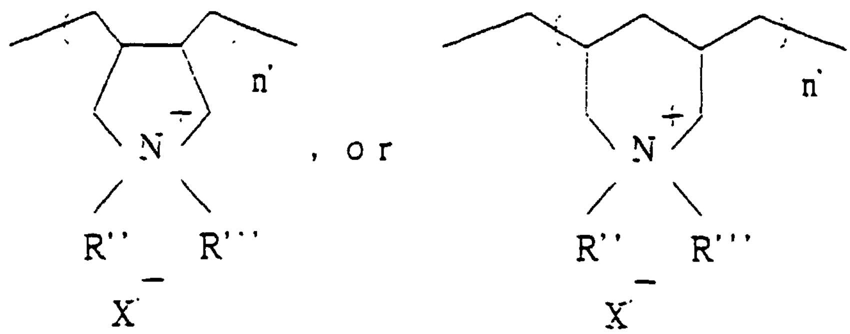

······ (13)

where each of R'' and R''' independently represents a hydrogen atom, a hydrocarbon group having 1-5 carbon atoms, $X'^-$ represents a paired anion, such as a halide ion, an $OH^-$ ion, a $CH_2=CHCOO^-$ ion, a $HCO_3^-$ ion, a carboxylic acid ion, a molybdic acid ion, a tungstic acid ion, a metavanadic acid ion, a pyrovanadic acid ion, a hydrogen pyrovanadic acid ion, and a hydrogen sulfite ion, and n' represents a natural number. However, the crosslinked polymer is not especially limited as long as it has a 3-D net structure insoluble to the solvent. The kinds of the functional group other than the cyclic quaternary ammonium group included in the crosslinked polymer are not especially limited, either.

The crosslinked polymer main body in General Formula (9) above means the main chain of the crosslinked polymer, and can be obtained either by polymerizing a monomer having a monovalent group of the cyclic amine structure as the functional group alone, or copolymerizing the above monomer with another monomer copolymerizible with the above monomer.

By subjecting the crosslinked polymer of General Formula (9) above to the quaternary reaction using the

quaternary agent, the crosslinking macromolecule ② can be readily obtained. Known quaternary agents of various kinds can be used, examples of which include, but not limited to: alkyl halide, such as methyl chloride, methyl bromide, methyl iodide, and ethyl iodide. The reaction conditions of the quaternary reaction are not especially limited, either, and known reaction conditions can be adopted. Further, after the quaternary reaction, the ion exchange may be conducted, so that the halide ion as the paired anion can be replaced with another paired anion, such as an $OH^-$ ion, a $CH_2CHCOO$- ion, a $HCO_3^-$ ion, a carboxylic acid ion, a molybdic acid ion, a tungstic acid ion, a metavanadic acid ion, a pyrovanadic acid ion, a hydrogen pyrovanadic acid ion, and a hydrogen sulfite ion.

Alternatively, the crosslinking macromolecule ② of General Formula (13) above can be readily obtained by polymerizing the monomer component containing the monomer of General Formula (10) or (11) above.

The crosslinking macromolecule ①can be readily obtained by filtering the polymer obtained by the above polymerization reaction, washing the polymer with the solvent in a satisfactory manner, and drying the washed polymer using popular methods, such as an evaporator and drying under a reduced pressure. The crosslinking macromolecule ②can be readily obtained by filtering the polymer obtained by the above synthesis method, washing the polymer with the solvent in a satisfactory manner, and drying the washed polymer using popular methods, such as an evaporator and drying under a reduced pressure. The crosslinking macromolecule may be in the form of gel. Gel of the crosslinking macromolecule (hereinafter, referred to as the macromolecule gel) can be readily obtained by letting the crosslinking macromolecule absorb and withhold a solvent having affinity to the same. Alternatively, the macromolecule gel can be obtained directly by polymerizing the monomer component if the polymerization conditions are set adequately.

The solvent withheld in the macromolecule gel is not especially limited, and can be any solvent used for reactions using the macromolecule gel, or the active-hydrogen-containing compound. In other words, the macromolecule gel may be produced first and thence added to the reaction system, or the crosslinking macromolecule, which is not turned into gel, may be added and mixed with the reaction system first, and thence turned into gel in the reaction system by being swelled as it absorbs the solvent or active-hydrogen containing compound used in the reaction. In either case, when the macromolecule gel is used as the catalyst, the active hydrogen in the active-hydrogen- containing compound starts to activate explosively at the point the macromolecule gel was produced. Thus, in case that the macromolecule gel is used as the catalyst, it is preferable that the macromolecule gel has been already produced before it is added to the reaction system. As has been discussed, by adding the macromolecule gel to the reaction system including the active-hydrogen-containing compound followed by mixing, the active hydrogen in the active-hydrogen-containing compound which should be activated can be readily activated.

Because the macromolecule gel withholds the solvent or the active-hydrogen-containing compound in the internal thereof, it forms an intermediate between sold and liquid. Thus, the macromolecule gel can be readily separated and removed from the reaction system after the reaction. Also, the macromolecule gel can efficiently activate the active hydrogen at the active site on the surface and/or in the internal of the supporting structure which forms the gel. For example, in case that the macromolecule gel has the 3-D net structure, the macromolecule gel can activate the active hydrogen at the active site on the surface and/or in the internal of the 3-D net structure, thereby attaining a high degree of freedom. Consequently, not only can a reaction rate, but also a conversion ratio of the active-hydrogen-containing compound can be improved further.

The macromolecule gel can be directly used as the catalyst, but it can carry metal when occasion demands. Examples of metal include, but not limited to: copper, lead, nickel, zinc, iron, cobalt, chrome, manganese, bismuth, tin, antimony, alkaline earth metal, etc.

In case that the metal is carried by the macromolecule gel, a carried amount of the metal based on a weight of the crosslinking macromolecule is not especially limited, and can be set adequately depending on the kinds of the crosslinking polymer or the kinds of synthesis reactions adopted. Actions called as "carrying" in the present invention include chelating, salts formation, absorption, wrapping, etc. Thus, the form thereof is not especially limited. Also, metal can be carried in the form of either ions or metal (atoms). Examples of the form of ion include: oxide, halide, and sulfide, etc.

A method of letting the macromolecule gel carry the metal is not especially limited, and popular methods can be used. For example, to let the macromolecule gel carry the metal, such as lead, the macromolecule gel is soaked in an aqueous solution, in which a predetermined amount of lead compound, such as lead nitrate and lead acetate, has been dissolved, and the reaction solution is stirred under predetermined conditions to conduct cation exchange. Then, the macromolecule gel is removed by means of filtration or the like, and washed with water, whereby the metal is carried by the macromolecule gel.

The activating catalyst ①of the present invention is arranged in such a manner that the ESPD charge of at least one atom in the atomic group forming the functional group included in the catalyst is preferably in a range between -0.65 and -1.05 (electrons), more preferably in a range between -0.65 and -1.00, and most preferably in a range between -0.70 and -0.95. The activating catalyst ①having the atom showing the ESPD charge smaller than - 1.05 is not preferable, because, although the catalytic ability is improved, the selectivity is reduced. On the other hand, the activating catalyst ①having the atom showing the ESPD charge exceeding -0.65 is not preferable, because the catalytic

ability is reduced.

The activating catalyst ② of the present invention is arranged in such a manner that it includes a cyclic quaternary ammonium group as the functional group, and that the ESPD charge of a nitrogen atom forming the ammonium group is preferably in a range between +0.4 and +1.3 (electrons), more preferably in a range between +0.5 and +1.2, and most preferably in a range between +0.55 and +1.1. The activating catalyst ② having a nitrogen atoms showing the ESPD charge exceeding +1.3 is not preferable, because, although the catalytic ability is improved, the selectivity is reduced. On the other hand, the activating catalyst ② having the nitrogen atom showing the ESPD charge smaller than +0.4 is not preferable, because the catalytic ability is reduced.

Next, the following will explain the ESPD charge computed with the comprehensive molecule design aid system, commercially known as Spartan® (version 5.0) of Wavefunction, Inc. For ease of explanation, the following will explain a case where the activating catalyst ①has a functional group of a structure derived from a derivative of aminothiahexanoic acid or dimethylamine, and a case where the activating catalyst ② has a functional group of a structure derived from vinylpyrrolidines or vinylpiperidines. Also, for ease of explanation, examples of derivatives referred to hereinafter include derivatives which are judged as inappropriate as the activating catalyst of the present invention from the computed ESPD charge.

Initially, as to the activating catalyst ①, the following shows the computed ESPD charge for the structure derived from aminothiahexanoic acid. In the following, "N" forming a skeleton of aminothiahexanoic acid is denoted as skeleton N, for ease of explanation.

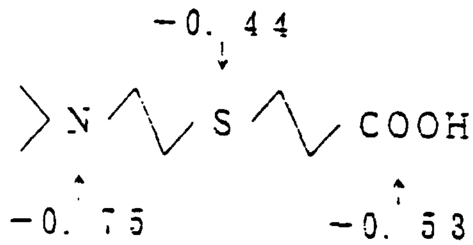

Next, the following will show the computed ESPD charges of structures derived from the aminothiahexanoic acid derivatives of various kinds:

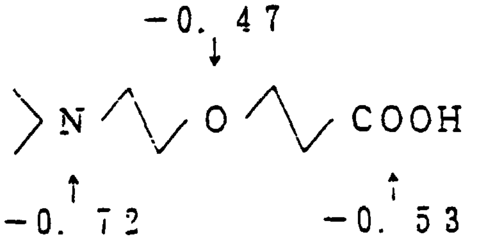
−0. 47
−0. 72
−0. 53

,

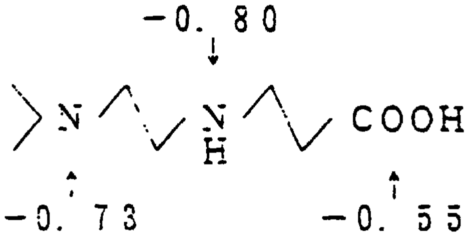
−0. 80
−0. 73
−0. 55

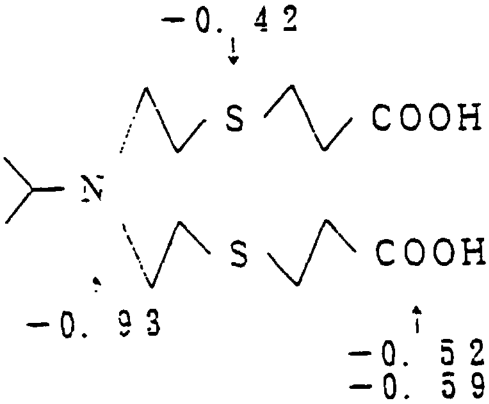
−0. 42
−0. 93
−0. 52
−0. 59

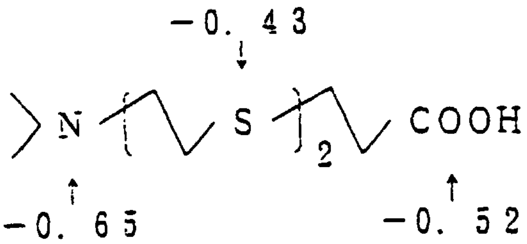
−0. 43
−0. 65
−0. 52

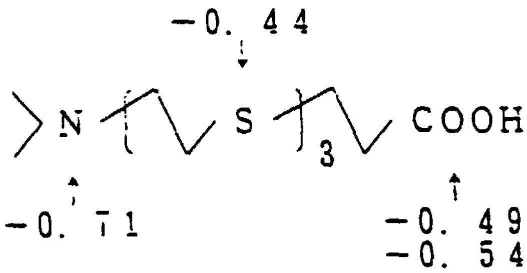
−0. 44
−0. 71
−0. 49
−0. 54

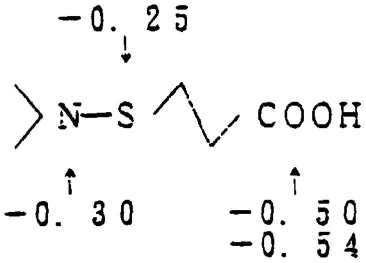
−0. 25
−0. 30
−0. 50
−0. 54
,
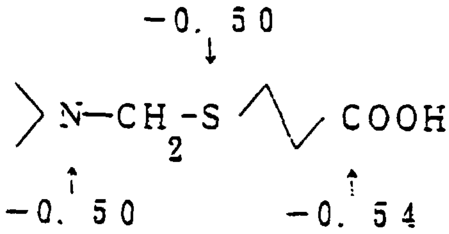
−0. 50
−0. 50
−0. 54

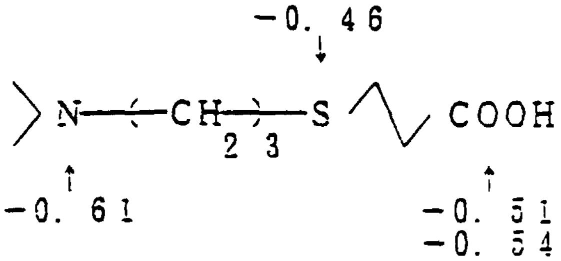
−0. 46
−0. 61
−0. 51
−0. 54

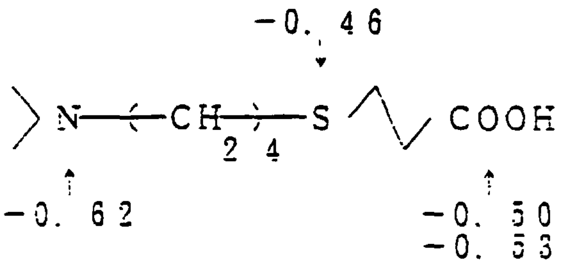
−0. 46
−0. 62
−0. 50
−0. 53

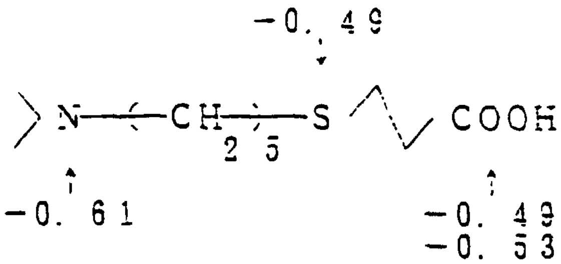
−0. 49
−0. 61
−0. 49
−0. 53

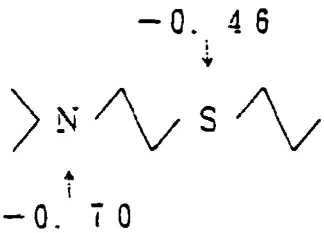
−0. 46
−0. 70

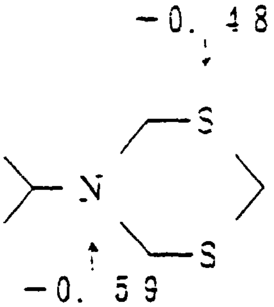


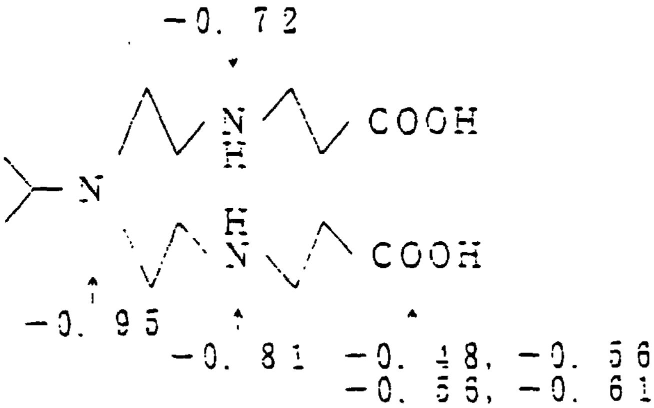


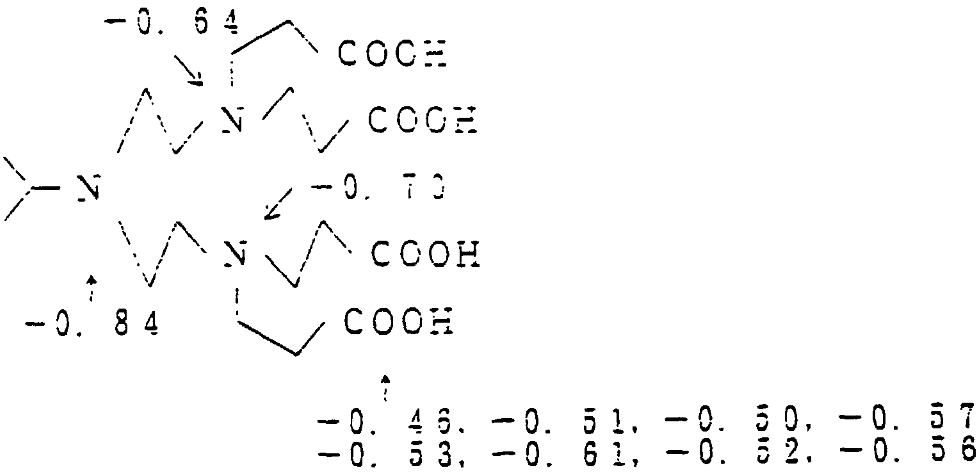


Likewise, the following will show the computed ESPD charge of the structure derived from dimethylamine. In the following, "N" forming the skeleton of dimethylamine is indicated as skeleton N, for ease of explanation:

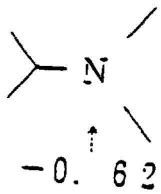

Next, the following will show the computed ESPD charges of the structures derived from dimethylamine derivatives of various kinds:

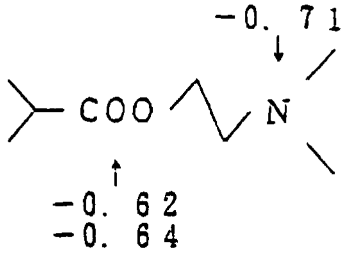

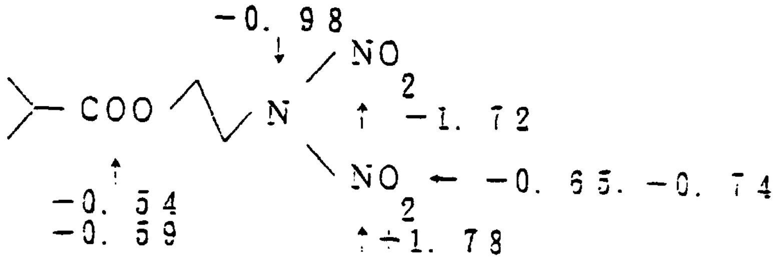

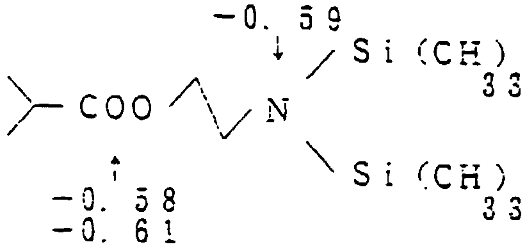

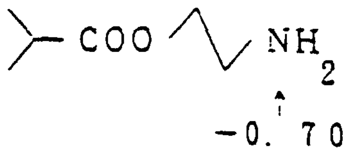 , 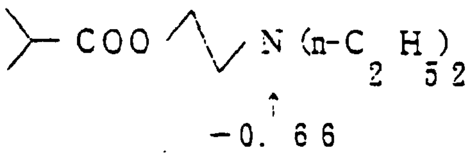

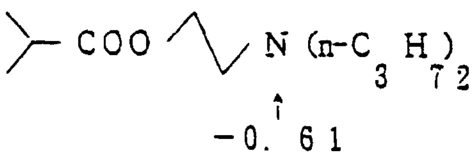

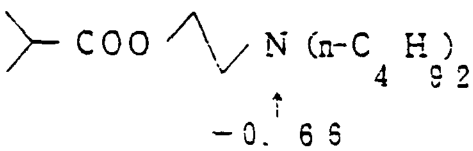

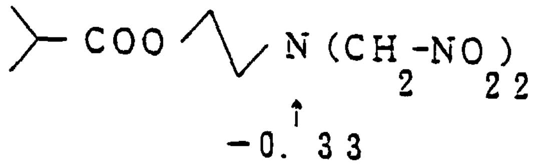
−0.33

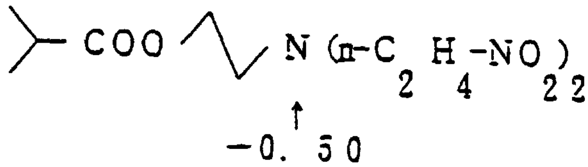
−0.50

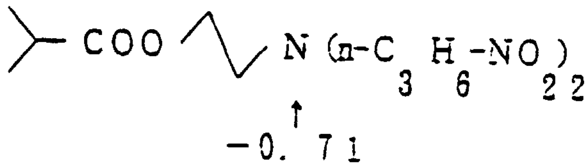
−0.71

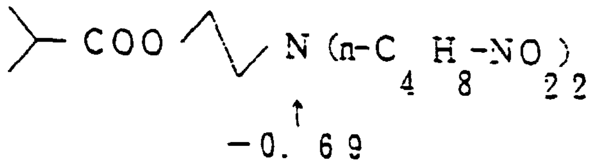
−0.69

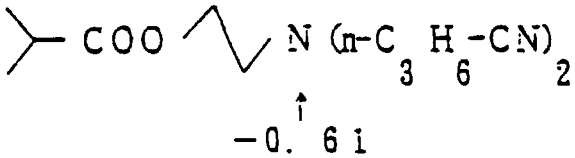
-0.61

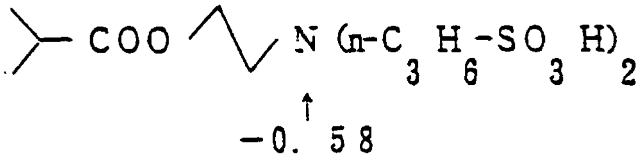
-0.58

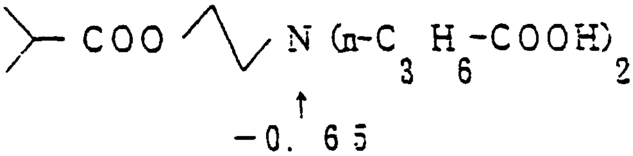
-0.65

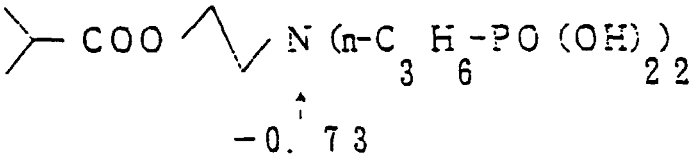
-0.73

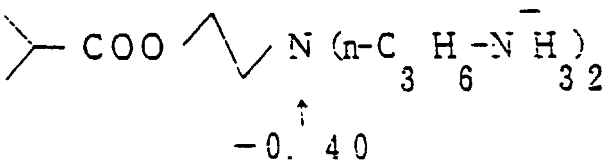
-0.40

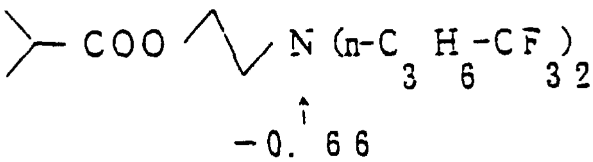
-0.66

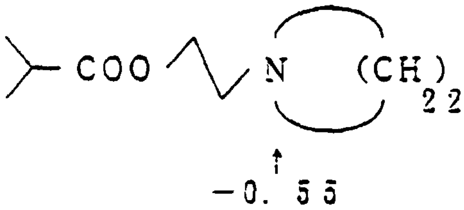

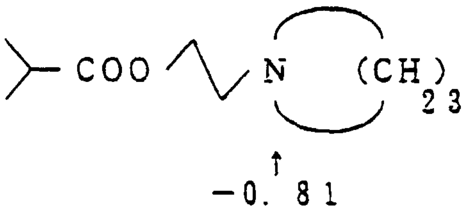

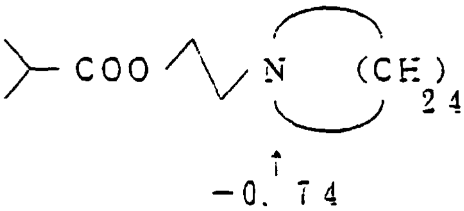

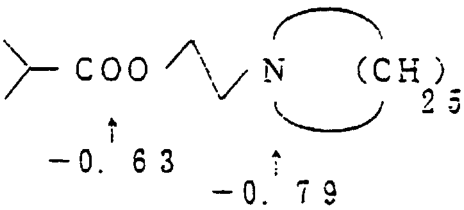

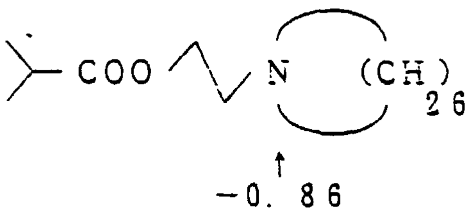

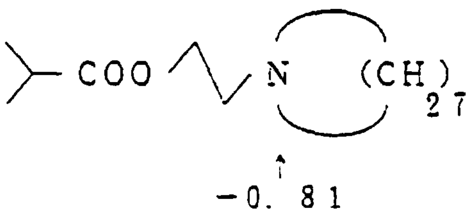

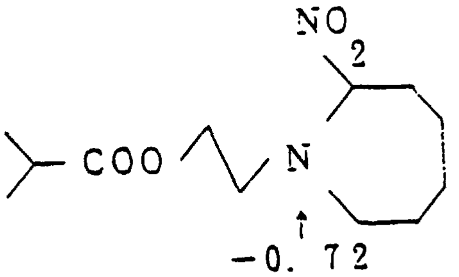

−0.72

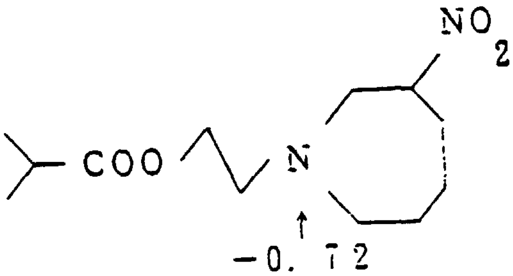

−0.72

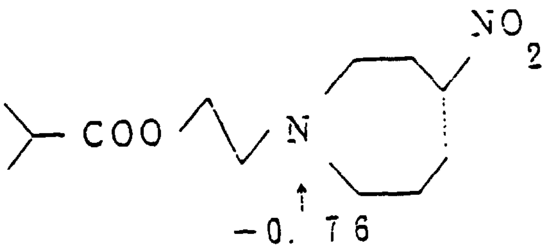

−0.76

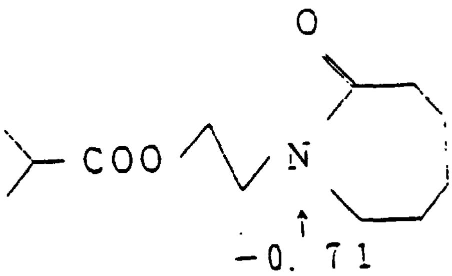

−0.71

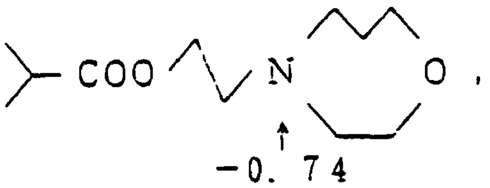

,
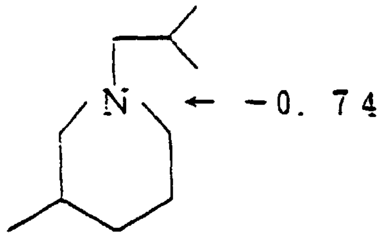


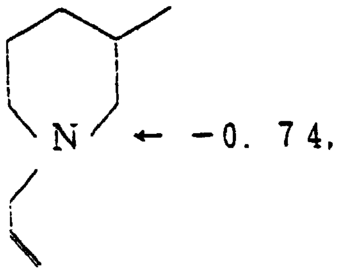

,
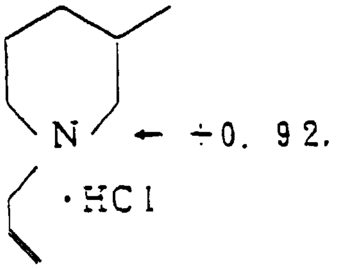

,
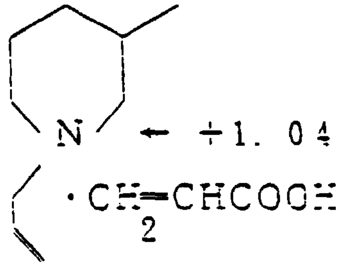


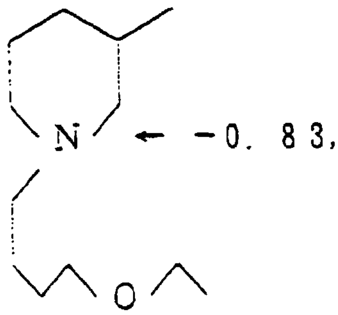

,
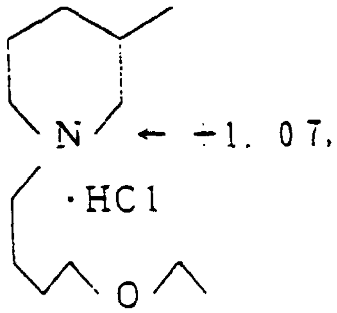

,
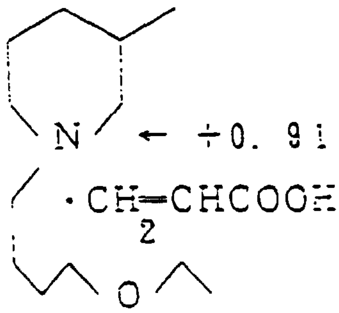


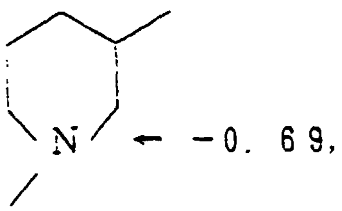

,
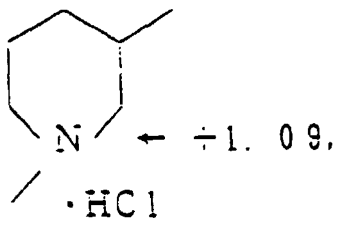

,
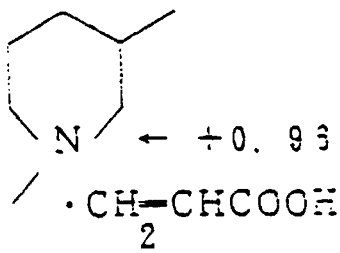


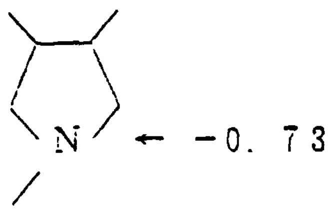


Also, the following will show the computed ESPD charge of lithium diisopropylamide (LDA) and that of lithium diethylamide as examples of a strong basic compound.

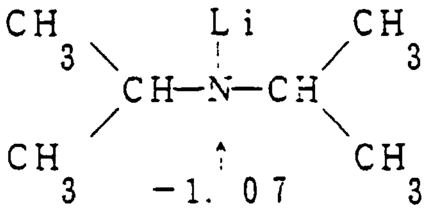

,

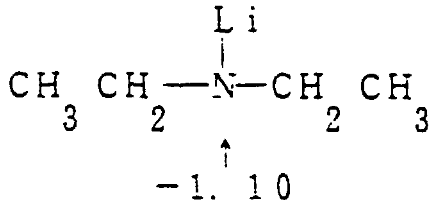

As has been discussed, the catalytic ability of the catalyst can be evaluated by computing the ESPD charge. In other words, the catalytic ability of the catalyst to activate the active hydrogen in the active-hydrogen-containing compound can be evaluated by the ESPD charge of the atom forming the functional group included in the catalyst.

Thus, suitable as the activating catalyst ①of the present invention is a catalyst, in which at least one atom in the atomic group forming the functional group has the ESPD charge in a range between -0.65 and -1.05 (electrons), more preferably in a range between -0.65 and -1.00, and most preferably in a range between -0.70 and -0.95. With the evaluating method of the present invention, by computing the ESPD charge of the atom forming the functional group included in the catalyst, it can be readily evaluated whether or not the catalyst has the active site, at which the catalyst efficiently activates the active hydrogen included in the compound. In short, the catalytic ability of the catalyst can be readily evaluated. A catalyst composed of atoms having the ESPD charges out of the above-specified preferred range is not suitable as the activating catalyst ①of the present invention, because such a catalyst does not have the active site, at which the active hydrogen is activated efficiently.

Suitable as the activating catalyst ①of the present invention is a catalyst, in which the atom showing the ESPD of the above-specified range is at least one kind of atom selected from the group consisting of a nitrogen atom, an oxygen atom, and a sulfur atom, in other words, the group consisting of the skeleton N, and heteroatoms of S, O and N(H). Further, more preferable as the activating catalyst ①of the present invention is a catalyst, in which the functional group containing the atom showing the ESPD charge of the above-specified range is a cyclic amino group. It should be appreciated that the functional group included in the activating catalyst ①is not limited to the functional group having a structure derived from the aminothiahexanoic acid derivative or dimethylamine derivative.

Next, as to the activating catalyst ②, the following shows the computed ESPD charges for the structures derived from vinyl pyrrolidines or vinylpiperidines.

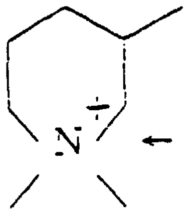

...... (a)

(paired anion)

$Cl^-$ ÷0. 93, $OH^-$ ÷0. 96

$Br^-$ ÷0. 95, $CH_2=CHCOO^-$ ÷0. 92

$I^-$ ÷0. 63

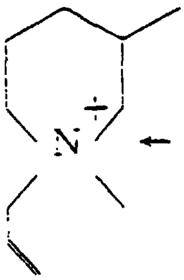

...... (b)

(paired anion)

$Cl^-$ ÷1. 05, $OH^-$ ÷0. 98

$Br^-$ ÷0. 85, $CH_2=CHCOO^-$ ÷1. 07

$I^-$ ÷0. 84

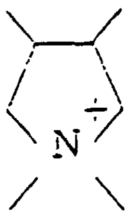

...... (c)

(paired anion)

$Cl^-$ ÷0. 93, $OH^-$ ÷0. 85

$I^-$ ÷0. 59 $CH_2=CHCOO^-$ ÷0. 86

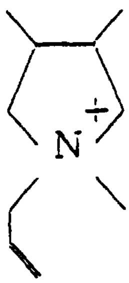

...... (d)

(paired anion)

$I^-$ ÷0. 59, $CH_2=CHCOO^-$ ÷0. 83

In case that the paired anion is a halide ion, the ESPD charge is assumed to decrease in order of $Cl^-$, $Br^-$, and $I^-$. For purpose of comparison, the following will show the computed ESPD charges of structures of the known ion-exchange resin.

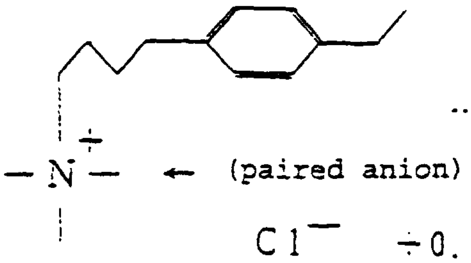


······ (e)

$Cl^-$ ÷0.92, $OH^-$ ÷1.10

$Br^-$ ÷0.83, $CH_2{=}CHCOO^-$ ÷1.00

$I^-$ ÷0.69

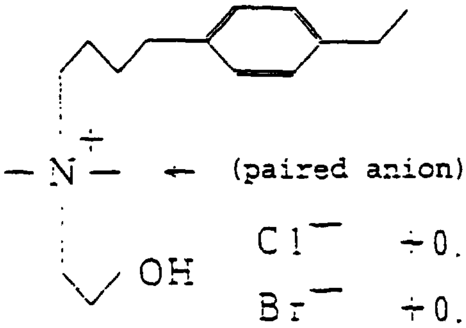


$Cl^-$ ÷0.98, $OH^-$ ÷0.96

$Br^-$ ÷0.89, $CH_2{=}CHCOO^-$ ÷1.13

$I^-$ ÷0.78

As has been discussed, by computing the ESPD charge, the catalytic ability of the catalyst can be evaluated. In other words, the catalytic ability of the catalyst to activate the active hydrogen in the active-hydrogen-containing compound can be evaluated by the ESPD charge of a nitrogen atom forming the cyclic quaternary ammonium group.

Thus, suitable as the activating catalyst ② of the present invention is a catalyst, in which the cyclic quaternary ammonium group is included as the functional group, and the ESPD charge of a nitrogen atom forming the ammonium group is in a range between +0.4 and +1.3 (electrons), more preferably in a range between +0.50 and +1.2, and most preferably in a range between +0.55 and +1.1. With the evaluating method of the present invention, by computing the ESPD charge of a nitrogen atom, it can be readily evaluated whether or not the catalyst has the active site, at which the catalyst efficiently activates the active hydrogen included in the compound. In short, the catalytic ability of the catalyst can be readily evaluated. A catalyst composed of atoms having the ESPD charges out of the above-specified preferred range is not suitable as the activating catalyst ② of the present invention, because such a catalyst does not have the active site, at which the active hydrogen is activated efficiently.

Also, more suitable as the activating catalyst ② of the present invention is a catalyst having the cyclic quaternary ammonium group composed of 5 or 6 members. It should be appreciated, however, that the functional group included in the activating catalyst ② is not limited to the functional group having a structure derived from vinylpyrrolidines or vinylpiperidines.

In the present invention, the active hydrogen activated by the activating catalyst mean, of all the hydrogen atoms included in a compound, only those participate in a desired reaction. Thus, the active hydrogen is not especially limited, but preferably has reactivity higher than that of a hydrogen atom directly bonded to a carbon atom of a heteroatom-free organic compound. Examples of the active hydrogen include:

a hydrogen atom directly bonded to a heteroatom;
a hydrogen atom (α-hydrogen atom) bonded to a carbon atom adjacent to an electron attracting group;
a hydrogen atom forming a substituent aromatic group;
a hydrogen atom forming a functional group, such as aldehyde and carboxylic acid; etc.

Also, examples of the hydrogen atom directly bonded to the heteroatom include: a hydrogen atom forming functional groups, such as a $-NH_2$ group, a -NH- group, a -CONH- group, a -OH group, and a -SH group. Further, examples of the hydrogen atom bonded to the carbon atom adjacent to the electron attracting groups include a hydrogen atom at the a-position of a carbonyl compound.

Therefore, the active-hydrogen-containing compound of the present invention means a compound containing any of the foregoing active hydrogens. Of all kinds of the active-hydrogen-containing compounds, the compound having at least one functional group selected from the group consisting of a -OH group, a -NH- group, and a-COOH group is more preferable than the others, and at least one kind of compound selected from the group consisting of unsaturated carboxylic acid, alcohol, and water is further preferable. Here, (meth)acrylic acid is most preferred as the unsaturated carboxylic acid. The active-hydrogen-containing compound may include more than one active hydrogen. In case that the active-hydrogen-containing compound has more than one active hydrogen, each of the active hydrogens may be either the same or different kind.

In the present invention, to activate the active hydrogen in the active-hydrogen-containing compound means to abstract the active hydrogen from the active-hydrogen-containing compound, or to facilitate dissociation thereof. In other words, the activating catalyst can be used suitably in various reactions involving nucleophilic addition either by abstracting the active hydrogen from the active-hydrogen-containing compound, or facilitating the dissociation thereof.

Next, the following will show examples of various reactions involving the activation of the active hydrogen in the active-hydrogen-containing compound, that is, reactions in which the activating catalyst can be suitably used. The following examples of reactions are a part of the reactions, to which the activating catalyst can be applied. Thus, it should be appreciated that the reactions to which the activating catalyst is applied are not limited to the following examples. In the reaction examples (reaction formulas), each of R, $R^1$, $R^2$, and $R^3$ independently represents an alkyl group or a hydrogen atom, Ar represents an aryl group, X represents a halide atom, such as F, Cl, and Br, and I, and A represents O, S, or NH.

A reaction related to the hydrogen atom directly bonded to the heteroatom is, for example, an addition reaction of a cyclic heterocompound (for example, ethylene oxide, ethylene imine, ethylene sulfide, etc.) to ammonia or amines (primary amines or secondary amines), expressed by the following reaction example (reaction formula):

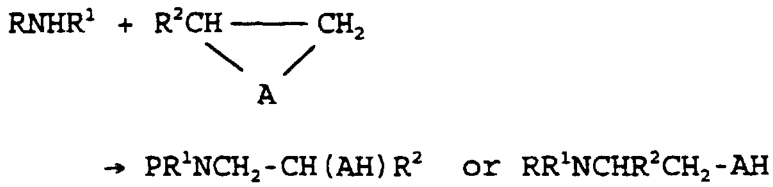


a conversion reaction of amines to amide, expressed by the following reaction examples:

$$R^1R^2NH + R^3COCl \rightarrow R^3CONR^1R^2 \quad \text{(N-substitution amide)}$$

$$R^1R^2NH + ArSO_2\text{-}Cl \rightarrow ArSO_2\text{-}NR^1R^2 \quad \text{(N-substitution sulfonamide)}$$

$$C_6H_5\text{-}NH_2 + (CH_3\text{-}CO)_2O \rightarrow C_6H_5\text{-}NHCOCH_3$$

$$C_6H_5\text{-}NH_2 + C_6H_5\text{-}SO_2\text{-}Cl \rightarrow C_6H_5\text{-}NHSO_2\text{-}C_6H_5$$

$$C_2H_5(CH_3)NH + C_6H_5\text{-}COCl \rightarrow C_6H_5\text{-}CON(CH_3)C_2H_5$$

$$C_2H_5(CH_3)NH + p\text{-}CH_3\text{-}C_6H_4\text{-}SO_2\text{-}Cl \rightarrow p\text{-}CH_3\text{-}C_6H_4\text{-}SO_2\text{-}N(CH_3)C_2H_5$$

a hydrolysis reaction of amides, expressed by the following reaction example:

$$RCONH_2 + H_2O \rightarrow RCOO^- + NH^+{}_4$$

an addition reaction of a cyclic heterocompound to amides, expressed by the following reaction example:

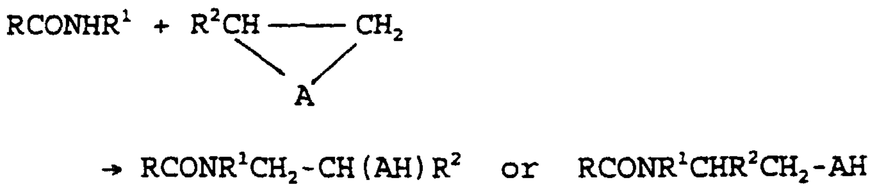

an addition reaction of a cyclic heterocompound to thioamides, expressed by the following reaction example:

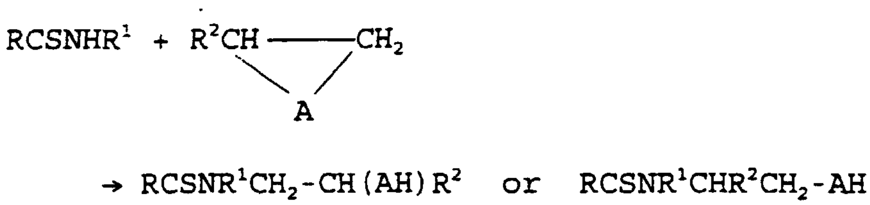

an addition of a cyclic heterocompound to water or alcohols (primary alcohols, secondary alcohols, or tertiary alcohols), expressed by the following reaction example:

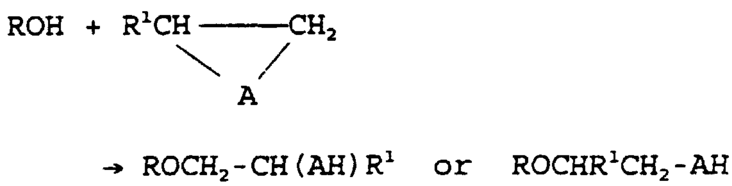

a reaction of alcohols and hydrogen halide, more specifically, the reaction of synthesizing isopropyl bromide from isopropyl alcohol and rich hydrogen bromide, expressed by the following reaction example:

$$ROH + HX \rightarrow RX + H_2O$$

$$(CH_3)_2CHOH + HBr \rightarrow (CH_3)_2CHBr + H_2O$$

a synthesis reaction of ester using alcohols, expressed by the following reaction example:

$ROH + p\text{-}CH_3\text{-}C_6H_4\text{-}SO_2\text{-}Cl$ (p-toluene sulfonyl chloride) $\rightarrow p\text{-}CH_3\text{-}C_6H_4\text{-}SO_2\text{-}OR$ (alkyltosylate) $ROH + R^1COOH \rightarrow R^1COOR + H_2O$

an oxidizing reaction of alcohols, expressed by the following reaction example:

$$RR^1CHOH + (1/2)O_2 \rightarrow RR^1CO + H_2O$$

an addition reaction of a cyclic heterocompoud to phenols, expressed by the following reaction example:

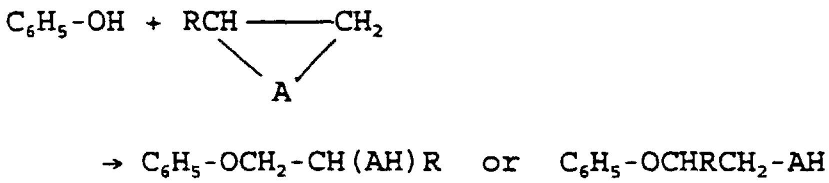

a reaction of synthesizing ethers from phenols and alkyl halide (Williamson synthesis reaction), more specifically, for example, the reaction of synthesizing p-nitrobenzyl-p-trylether from p-cresol and p-nitrobenzyl bromide, expressed by the following reaction example:

$$C_6H_5\text{-}OH + RI \rightarrow C_6H_5\text{-}OR$$

p-$CH_3$-$C_6H_4$-OH (p-cresol) + p-$BrCH_2$-$C_6H_4$-$NO_2$ (p-nitrobenzyl bromide) $\rightarrow$ p-$CH_3$-$C_6H_4$-O-(p-$CH_2$-$C_6H_4$-$NO_2$) (p-nitrobenzyl-p-trylether)

a synthesis reaction of ester using phenols, more specifically, for example, the reaction of synthesizing p-nitrophenyl acetate from p-nitrophenol and acetic anhydride, or the reaction of synthesizing o-bromophenyl-p-toluene sulfonic acid from o-bromophenol and p-toluene sulfonyl chloride, expressed by the following reaction:

$$C_6H_5\text{-}OH + RCOCl \rightarrow RCOOC_6H_5$$

p-$NO_2$-$C_6H_4$-OH (p-nitrophenol) + ($CH_3$-CO)$_2$O (acetic anhydride) $\rightarrow$ p-$NO_2$-$C_6H_4$-$OCOCH_3$ (p-nitrophenyl acetate)

o-Br-$C_6H_4$-OH (o-bromophenol) + p-$CH_3$-$C_6H_4$-$SO_2$-Cl (p-toluene sulfonyl chloride) $\rightarrow$ p-$CH_3$-$C_6H_4$-$SO_2$-O-(o-Br-$C_6H_4$) (o-bromophenyl-p-toluene sulfonic acid)

an addition reaction of a cyclic heterocompound to thiols (primary thiols, secondary thiols, or tertiary thiols), expressed by the following reaction example:

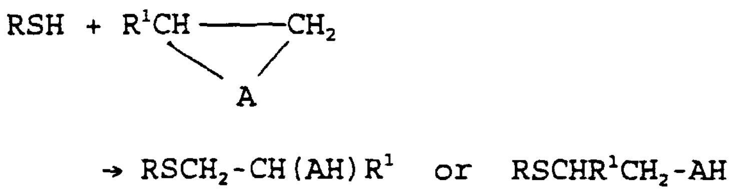

and an addition reaction of a cyclic heterocompound to thiophenols, expressed by the following reaction example:

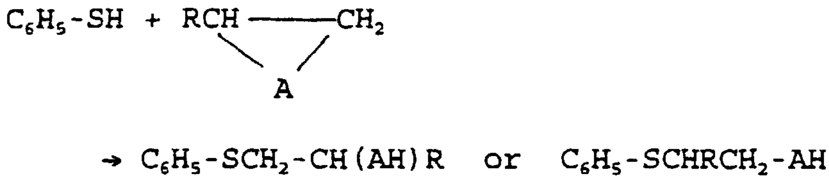

It should be appreciated, however, that reactions related to the hydrogen atom directly bonded to the heteroatom are not limited to the foregoing.

Also, examples of reactions related to the hydrogen atom bonded to the carbon atom adjacent to the electron attracting group include,

a halide reaction of ketones, more specifically, the reaction of introducing a bromine atom to cyclohexanone, expressed by the following reaction example:

$$-CHCO- + X_2 \rightarrow -CXCO- + HX$$

$$C_5H_{10}C = O + Br_2 \text{ (cyclohexane)} \rightarrow C_5H_9(Br)C = O + HBr$$

an aldol condensation reaction (more specifically, the reaction of synthesizing 3-hydroxybutanal from acetoaldehyde), expressed by the following reaction example:

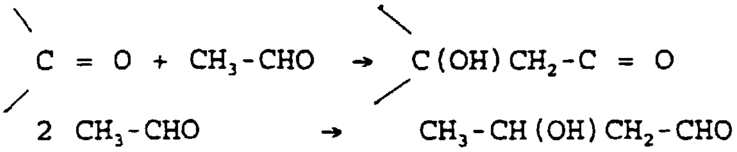

(acetoaldehyde) (3-hydroxybutanal)

a Perkin condensation reaction, expressed by the following reaction example:

$$C_6H_5\text{-}CHO + (CH_3\text{-}CO)_2O \rightarrow C_6H_5\text{-}CH = CHCOOH$$

a Knoevenagel condensation reaction, expressed by the following reaction example:

$$C_6H_5\text{-}CHO + CH_2(COOC_2H_5)_2 \rightarrow C_6H_5\text{-}CH = C(COOC_2H_5)_2$$

a Cope reaction, expressed by the following reaction example:

$$C_5H_{10}\text{-}C = O + N \equiv C\text{-}CH_2\text{-}COOC_2H_5 \rightarrow C_5H_{10}\text{-}C = C(CN)COOC_2H_5$$

nucleophic addition reactions of various kinds to carbonyl compounds (ketones), such as a Wittig reaction, expressed by the following reaction example:

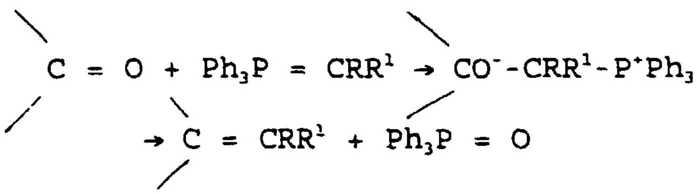

$$(C_6H_5)_2CO + Ph_3P = CH_2 \rightarrow (C_6H_5)_2CO^-\text{-}CH_2\text{-}P^+Ph_3 \rightarrow (C_6H_5)_2C = CH_2 + Ph_3P = O$$

a nucleophic acyl substitution reaction to ketones, such as a Claisen condensation reaction, expressed by the following reaction example:

$$-COOR + R^1CH_2\text{-}COOR^2 \rightarrow -CO\text{-}CH_2\text{-}COOR^2 + R^1OR$$

$$2\ CH_3\text{-}COOC_2H_5 \rightarrow CH_3\text{-}CO\text{-}CH_2\text{-}COOC_2H_5 + C_2H_5\text{-}OH$$

and an addition reaction (Michael addition reaction) to $\alpha,\beta$-unsaturated carbonyl compound (ketones), expressed by the following reaction:

$$CH_2 = CHCHO + H_2O \rightarrow CH_2(OH)CH_2\text{-}CHO$$

$$CH_2 = C(CH_3)COOC_2H_5 + N \equiv C\text{-}CH_2\text{-}COOC_2H_5 \rightarrow N \equiv C\text{-}CH\ (COOC_2H_5)\ CH_2\text{-}CH(CH_3)COOC_2H_5$$

It should be appreciated, however, that reactions related to the hydrogen atom bonded to the carbon atom adjacent to the electron attracting group are not limited to the foregoing.

Further, examples of reactions related to the hydrogen atom forming the substitution aromatic group include,

a Reimer-Tiemann reaction, expressed by the following reaction example:

$$C_6H_5\text{-}OH + CHCl_3 \rightarrow C_6H_4(CHCl_2)OH + HCl \rightarrow C_6H_4(OH)CHO \quad \text{(salicylaldehyde)}$$

and a Friedel-Crafts acylation reaction, expressed by the following reaction example:

$C_6H_4(OH)_2$ (resorcinol) + $CH_3(CH_2)_4$-COOH (caproic acid) $\rightarrow C_6H_3(OH)_2\text{-}CO(CH_2)_4\text{-}CH_3 + H_2O$ (2,4-dihydroxyphenyl-n-pentylketone)

It should be appreciated, however, that reactions related to the hydrogen atom forming the substitution aromatic group are not limited to the foregoing.

Examples of reactions related to the hydrogen atom forming the functional group, such as aldehyde and carboxylic acid, include,

an addition reaction of a cyclic heterocompound to carboxylic acids, expressed by the following reaction example:

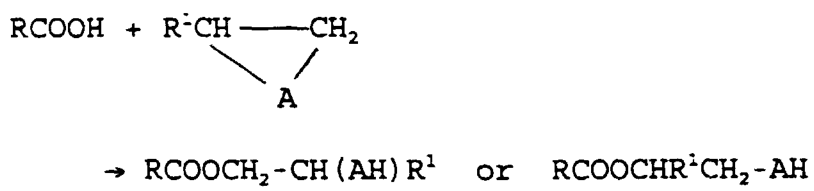

an addition reaction of a cyclic heterocompound to thiocarboxylic acids, expressed by the following reaction example:

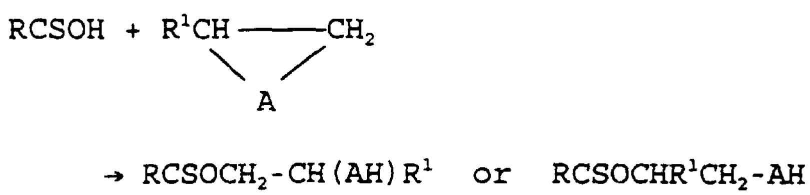

an addition reaction of alcohol to aldehydes, expressed by the following reaction example:

$$CH_3\text{-}CHO + 2C_2H_5\text{-}OH \rightarrow CH_3\text{-}CH(OC_2H_5)_2 + H_2O$$

a Cannizzaro reaction, expressed by the following reaction example:

$$2\ RCHO + H_2O \rightarrow RCH_2OH + RCOOH$$

It should be appreciated, however, that reactions related to the hydrogen atom forming the functional group, such as aldehyde and carboxylic acid, are not limited to the foregoing.

As has been discussed, the activating catalyst of the present invention can be suitably provided as a catalyst for various kinds of reactions involving the activation of an active hydrogen in the active-hydrogen-containing compound. Here, a method of separating and removing the activating catalyst from the reaction system is not especially limited. For example, the activating catalyst can be readily separated and removed from the reaction system by means of filtration and the like.

As has been discussed, the activating catalyst ①of the present invention is arranged in such a manner that the ESPD charge of at least one atom in the atomic group forming the functional group included in the catalyst is in a range between -0.65 and -1.05 (electrons). According to the above arrangement, the catalyst can efficiently activate the active hydrogen included in a compound using the atom showing the ESPD charge in the above-specified range as the active site.

Also, as has been discussed, the activating catalyst ② of the present invention is arranged in such a manner that it includes a cyclic quaternary ammonium group as the functional group, and that the ESPD charge of a nitrogen atom forming the ammonium group is in a range between +0.4 and +1.3 (electrons). According to the above arrangement, the catalyst can efficiently activate the active hydrogen included in a compound using the nitrogen showing the ESPD charge in the above-specified range as the active site.

In other words, these catalysts are suitable as activating catalysts for reactions of various kinds involving the activation of the active hydrogen. In addition, by using these activating catalysts, a reaction with excellent selectivity can be conducted. To be more specific, in case that the compound containing the active hydrogen is an unsaturated carboxylic acid, unsaturated carboxylic acid ester can be manufactured from the same. In case that the unsaturated carboxylic acid is (meth)acrylic acid, (meth)acrylic acid ester can be manufactured from the same.

As has been discussed, the method of activating the active hydrogen of the present invention is a method of activating the active hydrogen included in a compound using the above activating catalyst. Consequently, reactions of various kinds involving the activation of the active hydrogen can be conducted. In short, a reaction with excellent selectivity can be conducted.

Further, as has been discussed, the method of evaluating the activating catalyst of the present invention is a method of evaluating the catalytic ability of the catalyst to activate the active hydrogen included in a compound by the ESPD charge of an atom forming the functional group included in the catalyst (the atom includes a nitrogen atom forming the cyclic quaternary ammonium of the activating catalyst ②). Consequently, for example, by computing the ESPD charge of the atom forming the functional group included in the catalyst, it can be readily evaluated whether or not the catalyst has an active site, at which the active hydrogen included in the compound is effectively activated. In short, the catalytic ability of the catalyst can be readily evaluated.

In the following, the present invention will be explained in detail by way of examples and comparative examples without any intention to limit the scope thereof. In the following, a value computed using Spartan® (version 5.0) were used as the ESPD charge. However, in case of the activating catalyst ①, the smallest value (the largest negative value) among the values computed by Spartan® (version 5.0) was used as the ESPD charge (hereinafter, referred to as the min-ESPD charge). Also, the conversion ratio of the active-hydrogen-containing compound used as the reaction substrate, and the selectivity of the reaction product were computed in accordance with the following equations, respectively:

conversion ratio of active-hydrogen-containing compound
(%) = (molar number of consumed active-hydrogen-containing
compound/molar number of supplied active-hydrogen-containing compound) × 100

selectivity of reaction product (%) = (molar number of
active-hydrogen-containing compound converted to reaction
product/molar number of consumed active-hydrogen-containing compound) × 100.

(Example 1)

Polymer (hereinafter, referred to as NSA polymer) having a functional group of a structure derived from aminothiahexanoic acid was synthesized in the following manner. That is, 8 kg of cyclohexane as a solvent and 60 g of a predetermined kind of suspension agent were charged to and stirred in a reaction vessel equipped with a thermometer, a stirrer, a dropping device, and a reflux condenser. Then, 597 g of a monomer component made of 6-(N,N-dipropenyl amino)-4-thiahexaonoic acid and acrylic acid, 56 g of trimethylol propane triacrylate as a crosslinking monomer (monomer component), and 751 g of water as a solvent were added to the reaction vessel, and the resulting mixed solution was heated to 70°C. Here, a ratio of 6-(N,N-dipropenyl amino)-4-thiahexanoic acid to the monomer component was 5 mol%.

Meanwhile, 200 ml of 5 wt% aqueous solution of 2,2'-azobis-(2-amidinopropane)dihydrochloride (a polymerization initiator commercially known as V-50 of Wako Pure Chemical Industries, Ltd.) was charged to the dropping device.

Then, the aqueous solution in the dropping device was dropped to the mixed solution over 20 minutes. Then, the reaction solution in the reaction vessel was stirred constantly, and the stirring was stopped after 3 hours. Then, the reaction solution was allowed to stand overnight at room temperature so as to mature the reaction, whereby beads of polymer (polymer beads) having a particle size of 0.1-2 mm were synthesized.

The polymer beads were taken out and placed in a rotary evaporator. After cyclohexane was distilled out, the polymer beads were dried under a reduced pressure at 80°C, whereby 540 g of polymer beads were obtained. The resulting polymer had a functional group having a structure derived from aminothiahexanoic acid.

Then, a hydroxy propylation reaction of acrylic acid, which is an addition reaction of a cyclic heterocompound to carboxylic acids (unsaturated carboxylic acid), was conducted using the NSA polymer thus synthesized as the activating catalyst ①. The min-ESPD charge of the NSA polymer was -0.75 (electrons).

More specifically, acrylic acid (active-hydrogen-containing compound) and propylene oxide were charged to a reaction vessel equipped with a thermometer, a stirrer, etc. in such a manner that a charged amount of the latter was 1.2 time as much as that of the former in mole ratio, whereby a reaction solution was produced. Then, 10 wt% of the activating catalyst ①based on a weight of acrylic acid was added to the reaction solution. Then, the reaction solution was subjected to a reaction for 2 hours at 90°C with stirring, whereby hydroxy propylation of acrylic acid was completed.

When the reaction ended, the reaction solution was filtered, and the filtrate was analyzed by means of GC. Consequently, the conversion ratio of acrylic acid was 95% and the selectivity of hydroxy propyl acrylate (reaction product) was 92%, while the selectivity of dipropylene glycol monoacrylate (by-product of the side reaction) was 8%.

(Example 2)

A reaction and a manipulation were conducted in the same manner as Example 1 except that a polymer having a functional group of a structure derived from an aminothiahexanoic acid derivative with 2 units each having a carboxyl group was used as the activating catalyst ①, and that 3.6 wt% of the activating catalyst ①based on a weight of acrylic acid were added. The min-ESPD charge of the polymer was -0.93 (electrons).

Consequently, the conversion ratio of acrylic acid was 95%, and the selectivity of hydroxy propyl acrylate was 84%, while the selectivity of dipropylene glycol monoacrylate was 4.1%.

(Example 3)

The activating catalyst ①was manufactured in the following manner. That is, an aqueous solution containing a monomer (triallylamine hydrochloride) was prepared by means of neutralization by adding hydrochloric acid (36 wt% aqueous solution) to triallylamine. Then, 0.5 wt% of 2,2'-azobis-(2-amidinopropane)dihydrochloride serving as a polymerization initiator was added to the aqueous solution, whereby a water phase was formed. Meanwhile, toluene as a solvent and 1,1,1-trichloroethane were mixed with each other in a volume ratio of 1:1. Then, 1 wt% of sorbitan monostearate as a suspension agent and 1 wt% of polyvinyl alcohol based on a weight of the mixed liquid were added, whereby an oil phase was formed.

Then, predetermined amounts of the water phase and oil phase in a volume ratio of 1:4 were charged to a separable flask (reaction vessel) having a capacity of 1 L and equipped with a thermometer, a stirrer, and a reflux condenser. The monomer in the two phases forming a reaction liquid were subjected to polymerization for 4 hours at 55°C, and further 2 hours at 75°C with gradual mixing and stirring by turning the stirring blades at 200 rpm. Consequently, a polymer having a structure expressed by General Formula (A) was obtained:

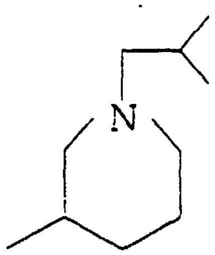

...... (A)

Subsequently, the reaction liquid was cooled, and polymer beads (solid) thus produced were taken out by means of filtration. Subsequently, the polymer beads were dried at 70°C under a reduced pressure. Consequently, a copolymer (hereinafter, referred to as TAA polymer) having a structure expressed by General Formula (A) above was obtained as the activating catalyst ①. The min-ESPD charge of the TAA polymer was -0.74 (electrons).

Then, a reaction and a manipulation were conducted in the same manner as Example 1 except that the TAA polymer was used as the activating catalyst ①, and that 10 wt% of the activating catalyst ①based on a weight of acrylic acid was added.

Consequently, the conversion ratio of acrylic acid was 98%, and the selectivity of hydroxy propyl acrylate was 94%, while the selectivity of dipropylene glycol monoacrylate was 5.0%. In addition, the selectivity of propylene glycol diacrylate (by-product of the side reaction) was 0.08%.

(Comparative Example 1)

Here, a reaction and a manipulation were conducted in the same manner as Example 1 except that dimethyl allylamine was used as the comparative activating catalyst ①. The min-ESPD charge of dimethyl allylamine was -0.62 (electrons), which did not fall in the preferred range of the present invention.

Consequently, the conversion ratio of acrylic acid was 76%, and the selectivity of hydroxy propyl acrylate was 87%, while the selectivity of dipropylene glycol monoacrylate was 13.6%. In addition, the selectivity of propylene glycol diacrylate was 0.10%. The foregoing results reveal that when the comparative activating catalyst ①was used, the conversion ratio of acrylic acid was reduced.

(Comparative Example 2)

Here, a reactions and a manipulation were conducted in the same manner as Example 1 except that triallylamine was used as the comparative activating catalyst ①. The min-ESPD charge of triallylamine was -0.54 (electrons), which did not fall in the preferred range of the present invention.

Consequently, the conversion ratio of acrylic acid was 75%, and the selectivity of hydroxy propyl acrylate was 83%, while the selectivity of dipropylene glycol monoacrylate was 15.4%. In addition, the selectivity of propylene glycol diacrylate was 0.10%. The foregoing results reveal that when the comparative activating catalyst ①was used, the conversion ratio of acrylic acid was reduced.

(Comparative Example 3)

Here, a reaction and a manipulation were conducted in the same manner as Example 1 except that lithium diisopropyl amide (LDA) was used as the comparative activating catalyst ①. The min-ESPD charge of lithium diisopropyl amide was -1.07 (electrons), which did not fall in the preferred range of the present invention.

In this reaction, the side reaction proceeded dominantly, and the target product of hydroxy propyl acrylate was hardly obtained.

(Example 4)

A hydroxy ethylation reaction of acrylic acid was conducted by using the NSA polymer used in Example 1 (min-ESPD charge = -0.75 (electrons)) as the activating catalyst ①. To be more specific, a predetermined amount of acrylic acid was charged to a reaction vessel equipped with a thermometer, a gas conduit, a stirrer, etc. Also 10 wt% of the activating catalyst ①based on a weight of acrylic acid was added. Then, acrylic acid was heated to 70°C with stirring, after which ethylene oxide was constantly introduced to the reaction vessel through the gas conduit over 4 hours

in such a manner that a charged amount of ethylene oxide was 1.1 time as much as that of acrylic acid in mole ratio. Then, the reaction solution was matured for 3 hours at 70°C with stirring, whereby the hydroxy ethylation of acrylic acid was completed. When the reaction ended, the reaction solution was filtered and the filtrate was analyzed by means of GC. Consequently, the conversion ratio of acrylic acid was 90%, and the selectivity of hydroxy ethyl acrylate (reaction product) was 91%, while the selectivity of diethylene glycol monoacrylate (by-product of the side reaction) was 7%.

(Example 5)

Here, the TAA polymer (min-ESPD charge = -0.74 (electrons)) used in Example 3 was used as the activating catalyst ①. More specifically, a predetermined amount of acrylic acid was charged to a reaction vessel equipped with a thermometer, a gas conduit, a stirrer, etc. Also, 10 wt% of the activating catalyst ①based on a weight of acrylic acid was added. Then, after acrylic acid was heated to 90°C with stirring, ethylene oxide was continuously introduced to the reaction vessel through the gas conduit over 3 hours in such a manner that a charged amount of ethylene oxide was 1.2 time as much as that of acrylic acid in mole ratio, whereby a reaction solution was prepared. Then, the reaction solution was matured for 2 hours at 90°C with stirring, whereby hydroxy ethylation of acrylic acid was completed.
Consequently, the conversion ratio of acrylic acid was 79%, and the selectivity of hydroxy ethyl acrylate was 94%, while the selectivity of diethylene glycol monoacrylate was 6.1%. In addition, the selectivity of ethylene glycol diacrylate was 0.09%.

(Example 6)

Here, a hydroxy propylation reaction of n-butyl alcohol, which is an addition reaction of a cyclic heterocompound to alcohols, was conducted using the NSA polymer used in Example 1 as the activating catalyst ①.
More specifically, n-butyl alcohol (active-hydrogen-containing compound) and propylene oxide were charged to a reaction vessel equipped with a thermometer, a stirrer, etc. in such a manner that a charged amount of the latter was 1.2 time as much as that of the former in mole ratio, whereby a reaction solution was prepared. Then, 10 wt% of the activating catalyst ①based on a weight of n-butyl alcohol was added to the reaction solution. Subsequently, the resulting reaction solution was subjected to a reaction for 4.5 hours at 90°C with stirring, whereby the hydroxy propylation of n-butyl alcohol was completed.
When the reaction ended, the reaction solution was filtered and the filtrate was analyzed by means of GC. Consequently, the conversion ratio of n-butyl alcohol was 67%, and the selectivity of an added body (reaction product), in which 1 mol of propylene oxide was added to n-butyl alcohol, was 86%, while the selectivity of an added body (by-product of the side reaction), in which 2 mol of propylene oxide was added to n-butyl alcohol, was 14%.

(Example 7)

Here, a hydroxy propylation reaction of phenol was conducted using the NSA polymer used in Example 1 as the activating catalyst ①. More specifically, phenol (active-hydrogen-containing compound) and propylene oxide were charged to a reaction vessel equipped with a thermometer, a stirrer, etc. in such a manner that a charged amount of the latter was 1.6 time as much as that of the former in mole ratio, whereby a reaction solution was prepared. Then, 10 wt% of the activating catalyst ①based on a weight of phenol was added to the reaction solution. Then, the resulting reaction solution was subjected to a reaction for 4.5 hours at 90°C with stirring, whereby the hydroxy propylation of phenol was completed.
When the reaction ended, the reaction solution was filtered and the filtrate was analyzed by means of GC. Consequently, the conversion ratio of phenol was 100%, and the selectivity of an added body (reaction product), in which 1 mol of propylene oxide was added to phenol, was 94%, while the selectivity of an added body (by-product of the side reaction), in which 2 mol of propylene oxide was added to phenol, was 5%.

(Example 8)

Here, a hydroxy propylation reaction of 2-pyrolidone, which is an addition reaction of a cyclic heterocompound to amides, was conducted using the NSA polymer used in Example 1 as the activating catalyst ①.
More specifically, 2-pyrolidone (active-hydrogen-containing compound) and propylene oxide were charged to a reaction vessel equipped with a thermometer, a stirrer, etc. in such a manner that a charged amount of the latter was 1.4 time as much as that of the former in mole ratio, whereby a reaction solution was prepared. Then, 10 wt% of the activating catalyst ①based on a weight of 2-pyrolidone was added to the reaction solution. Then, the resulting reaction solution was subjected to a reaction for 5 hours at 90°C with stirring, whereby the hydroxy propylation of 2-pyrolidone was completed.

When the reaction ended, the reaction solution was filtered and the filtrate was analyzed by means of GC. Consequently, the conversion ratio of 2-pyrolidone was 44%, and the selectivity of an added body (reaction product), in which 1 mol of propylene oxide was added to 2-pyrolidone, was 94%, while the selectivity of an added body (by-product of the side reaction), in which 2 mol of propylene oxide was added to 2-pyrolidone, was 2%.

(Comparative Example 4)

Here, a reaction and a manipulation were conducted in the same manner as Example 8 except that a commercially available ion-exchange resin known as IRA-410 of Rohm & Haas Co. was used as the comparative activating catalyst ①. The ion-exchange resin had a structure expressed by General Formula (B):

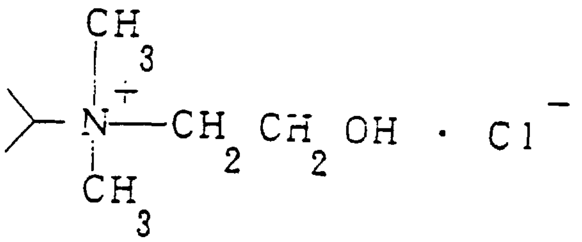

...... (B)

The min-ESPD charge of the ion-exchange resin was +0.89 (electrons). However, because it did not include a cyclic quaternary ammonium group, it did not fall in the preferred range of the present invention.

In the above reaction, the side reaction proceeded dominantly, and the target product of an addition body, in which 1 mol of propylene oxide was added to 2-pyrolidone, was hardly obtained.

(Comparative Example 5)

Here, a reaction and a manipulation were conducted in the same manner as Example 8 except that lithium diisopropyl amide was used as the comparative activating catalyst ①.

In the above reaction, the side reaction proceeded dominantly, and the target product of an addition body, in which 1 mol of propylene oxide was added to 2-pyrolidone, was hardly obtained.

(Example 9)

Here, a condensation reaction (Knoevenagel condensation reaction) of malonic acid and benzaldehyde, which is a reaction involving the hydrogen atom bonded to the carbon atom adjacent to the electron attracting group, was conducted using the NSA polymer used in Example 1 as the activating catalyst ①.

More specifically, malonic acid as the active-hydrogen-containing compound and benzaldehyde were charged to a reaction vessel equipped with a thermometer, a stirrer, etc. in such a manner that a mole ratio of a charged amount of the latter to that of the former is 1:1, whereby a reaction solution was prepared. Then, 10 wt% of the activating catalyst ①based on a weight of malonic acid was added to the reaction solution. Then, the resulting reaction solution was subjected to a reaction for 11 hours at 90°C with stirring, whereby the condensation reaction of malonic acid and benzaldehyde was completed.

When the reaction ended, the reaction solution was filtered and the filtrate was analyzed by means of GC. Consequently, the conversion ratio of malonic acid was 61%, and the selectivity of cinnamic acid as the reaction product was 84%.

(Example 10)

The activating catalyst ①was manufactured by conducting a reaction and a manipulation in the same manner as Example 3 except that an aqueous solution, which contains tertiary amine prepared by subjecting diallylamine to methylation as the monomer instead of triallyamine hydrochloride, was prepared. The min-ESPD charge of the activating catalyst ①was -0.73 (electrons).

Then, a hydroxy propylation reaction of acrylic acid was conducted using the activating catalyst ①. More specifically, acrylic acid and propylene oxide were charged to a reaction vessel equipped with a thermometer, a stirrer, etc. in such a manner that a charged amount of the latter was 1.24 time as much as that of the former in mole ratio, whereby a reaction solution was prepared. Then, 5 wt% of the activating catalyst ①based on a weight of acrylic acid

was added to the reaction solution. Then, the resulting reaction solution was subjected to a reaction for 4 hours at 70°C with stirring, whereby the hydroxy propylation of acrylic acid was completed.

Consequently, the conversion ratio of acrylic acid was 48.4%, and the selectivity of hydroxy propl acrylate was 66.3%, while the selectivity of dipropylene glycol monoacrylate was 21.9%. Also, the selectivity of propylene glycol diacrylate was 0.0%.

(Example 11)

A polymer (hereinafter, referred to as polymer bd-$I^-$) was synthesized in the manner specified below, which has a functional group having a structure derived from a piperidine ring expressed by General Formula (b) above with a paired anion of $I^-$, or a structure derived from a pyrrolidine ring expressed by General Formula (d) above with a paired anion of $I^-$.

Initially, N,N,N-triallylamine was neutralized with an equal amount of hydrochloric acid (36 wt% aqueous solution), whereby an aqueous solution of N,N,N-triallylamine hydrochloride was prepared as an aqueous solution containing the monomer. Then, 0.8 wt% of 2,2'-azobis-(2-amidinopropane)dihydrochloride serving as a polymerization initiator based on a weight of the aqueous solution was added to 1000 g of the aqueous solution of N,N,N-triallylamine hydrochloride, whereby a water phase was formed.

Meanwhile, 4 L of toluene and 4 L of 1,1,1-trichloroethane, both serving as dispersion media, were mixed with each other. Then, 0.8 wt% of sorbitan monostearate and 0.8 wt% of polyvinyl alcohol based on a weight of the mixed liquid, both serving as the suspension agents, were added to the mixed liquid, whereby an oil phase was formed.

Then, the water phase and oil phase were charged to a polymerization pot having a capacity of 20 L and equipped with a thermometer, a stirrer and a reflux condenser. The water and oil phases forming a reaction liquid were subjected to polymerization for 4 hours at 55°C, and further 2 hours at 85°C with gradual mixing and stirring by turning the stirring blade at 120 rpm.

Subsequently, the reaction solution was cooled, and polymer beads (solid) thus produced were taken out by means of filtration. Then, the polymer beads were dried at 60°C under a reduced pressure, whereby 259 g of granular resin (average particle size: 0.6mm) having the cyclic amine structure was obtained.

Then, a mixture of 118 g of a methyl alcohol solution of 12 wt% diethylene glycol divinyl ether and 14.7g of 7 wt% 2,2'-azobis-(2-amidino propane)dihydrochloride aqueous solution (polymerization initiator) was impregnated to 41 g of the resulting polymer. Then, the polymer which had impregnated the mixture was heated for 2.5 hours in 690 g of toluene with nitrogen being bubbled. The temperature of the toluene phase at this point was 67°C. After the reaction solution was cooled, the resulting reaction product was washed with toluene first and thence methyl alcohol, and dried at 60°C under a reduced pressure, whereby resin made of the polymer treated with diethylene glycol divinyl ether was obtained.

Then, 5 g of the dried resin was added to 50 g of methyl alcohol together with 15 g of methyl iodide as a quaternary agent, after which the reaction solution was treated for 4 hours at 45°C. Then, the solid in the reaction liquid was filtered out, and washed with methyl alcohol. Then, the solid was dried at 60°C under a reduced pressure, whereby spherical resin of the polymer bd-$I^-$ was obtained.

Then, a hydroxy ethylation reaction of acrylic acid, which is an addition reaction of a cyclic heterocompound to carboxylic acids (unsaturated carboxylic acid), was conducted using the polymer bd-$I^-$ thus synthesized as the activating catalyst ②. The ESPD charge of the polymer bd-$I^-$ was +0.84 (electrons) in a structure derived from a piperidine ring, and +0.59 in a structure derived from a pyrrolidine ring.

More specifically, a predetermined amount of acrylic acid (active-hydrogen-containing compound) was charged to a reaction vessel equipped with a thermometer, a gas conduit, a stirrer, etc., while 70 vol% of the activating catalyst ②based on a weight of acrylic acid was added. After acrylic acid was heated to 70°C with stirring, ethylene oxide was constantly introduced to the reaction vessel through the gas conduit over 1.5 hour in such a manner that a charged amount of ethylene oxide was 1.05 time as much as that of acrylic acid in mole ratio. Then, the reaction solution was subjected to maturation for 3.5 hours at 70°C with stirring, whereby hydroxy ethylation of acrylic acid was completed.

When the reaction ended, the reaction solution was filtered, and the filtrate was analyzed by means of GC. Consequently, the conversion ratio of acrylic acid was 94.7%, and the selectivity of hydroxy ethyl acrylate (reaction product) was 88.5%, while the selectivity of diethylene glycol monoacrylate (by-product of the side reaction) was 3.5%. Also, the selectivity of ethylene glycol diacrylate (by-product of the side reaction) was 0.22%.

(Example 12)

Here, a hydroxy propylation reaction of acrylic acid was conducted using the polymer bd-$I^-$ synthesized in the manner described in Example 11 as the activating catalyst ②. More specifically, acrylic acid and propylene oxide

were charged to a reaction vessel equipped with a thermometer, a stirrer, etc. in such a manner that a charged amount of the latter was 1.24 time as much as that of the former in mole ratio, whereby a reaction solution was prepared. Then, 5 wt% of the activating catalyst ②based on a weight of acrylic acid was added to the reaction solution, and the reaction solution was subjected to a reaction for 4 hours at 70°C with stirring, whereby the hydroxy propylation of acrylic acid was completed.

When the reaction ended, the reaction solution was filtered, and the filtrate was analyzed by means of GC. Consequently, the conversion ratio of acrylic acid was 59.7%, and the selectivity of hydroxy propyl acrylate (reaction product) was 81.9%, while the selectivity of dipropylene glycol monoacrylate (by-product of the side reaction) was 15.5%. Also, the selectivity of propylene glycol diacrylate (by-product of the side reaction) was 0.07%.

(Example 13)

A polymer (hereinafter, referred to as polymer ac-$Cl^-$ ) having a functional group of a structure derived from a piperidine ring expressed by General Formula (a) above with a paired anion of $Cl^-$, or of a structure derived from a pyrrolidine ring expressed by General Formula (c) above with a paired anion of $Cl^-$ was synthesized in the following manner.

That is, initially, a copolymer of diallyl dimethyl ammonium chloride and diallylamine were synthesized in a predetermined manner. Then, 3331 g of a 30 wt% aqueous solution of the copolymer was dispersed in 10 L of toluene, to which 25 g of ethyl cellosolve and 25 g of sorbitan monopalmitate had been added. Then, 91 g of epichlorohydorine was dropped to the dispersing solution over 1 hour. When the dropping ended, the reaction liquid was heated for 4 hours at 90°C. When the reaction liquid was cooled, the solid in the reaction solution was filtered out, and washed with methyl alcohol. Then, the solid was dried at 60°C under a reduced pressure, whereby spherical resin of the polymer ac-$Cl^-$ was obtained.

Then, a reaction and a manipulation were conducted in the same manner as Example 11 using the polymer ac-$Cl^-$ thus synthesized as the activating catalyst ②. The ESPD charge of the polymer ac-$Cl^-$ was +0.93 (electrons) in the structure derived from a piperidine ring, and +0.93 in the structure derived from a pyrrolidine ring.

Consequently, the conversion ratio of acrylic acid was 88.4%, and the selectivity of hydroxy ethyl acrylate was 85.2%, while the selectivity of diethylene glycol monoacrylate was 4.6%. Also, the selectivity of ethylene glycol diacrylate was 0.25%.

(Example 14)

Here, a reaction and a manipulation were conducted in the manner described in Example 12 using the polymer ac-$Cl^-$ synthesized in the manner described in Example 13 as the activating catalyst ②. Consequently, the conversion ratio of acrylic acid was 73.6%, and the selectivity of hydroxy propyl acrylate was 85.8%, while the selectivity of dipropylene glycol monoacrylate was 10.4%. In addition, the selectivity of propylene glycol diacrylate was 0.06%.

(Example 15)

A polymer (hereinafter, referred to as polymer ac-$OH^-$ ) having a functional group of a structure derived from a piperidine ring expressed by General Formula (a) above with a paired anion of $OH^-$, or a structure derived from a pyrrolidine ring expressed by General Formula (c) above with a paired anion of $OH^-$ was synthesized in the following manner.

That is, initially, a copolymer of diallyl dimethyl ammonium chloride and diallylamine were synthesized in a predetermined manner. Then, 3331 g of a 30 wt% aqueous solution of the copolymer was dispersed in 10 L of toluene, to which 25 g of ethyl cellosolve and 25 g of sorbitan monopalmitate had been added. Then, 91 g of epichlorohydorine was dropped to the dispersing liquid over 1 hour. When the dropping ended, the reaction liquid was heated for 4 hours at 90°C. When the reaction liquid was cooled, the solid in the reaction liquid was filtered out, and washed with methyl alcohol. Then, the solid was dried at 60°C under a reduced pressure, whereby spherical resin was obtained. Then, 200 g of the dried resin was added to 8512 ml of a 0.5N sodium hydroxide aqueous solution, and the reaction solution was stirred for 2 hours at room temperature. Further, the solid in the reaction solution was taken out, and added to 8512 ml of a 0.5N sodium hydroxide aqueous solution, after which the reaction solution was stirred for 2 hours at room temperature. Then, the solid was filtered out, and washed with ion-exchanged water until the pH of the washing solution reached 7. Then, the solid was washed with methyl alcohol and dried at 60°C under a reduced pressure, whereby spherical resin of the polymer ac-$OH^-$ was obtained.

Then, a reaction and a manipulation were conducted in the same manner as Example 11 using the polymer ac-$OH^-$ thus synthesized as the activating catalyst ②. The ESPD charge of the polymer ac-$OH^-$ was +0.96 (electrons) in the structure derived from a piperidine ring, and +0.85 in the structure derived from a pyrrolidine ring.

Consequently, the conversion ratio of acrylic acid was 92.0%, and the selectivity of hydroxy ethyl acrylate was 84.1%, while the selectivity of diethylene glycol monoacrylate was 4.3%. Also, the selectivity of ethylene glycol diacrylate was 0.32%.

(Example 16)

Here, a reaction and a manipulation were conducted in the same manner as Example 12 using the polymer ac-$OH^-$ synthesized in the manner described in Example 15 as the activating catalyst ②. Consequently, the conversion ratio of acrylic acid was 77.2%, and the selectivity of hydroxy propyl acrylate was 87.0%, while the selectivity of dipropylene glycol monoacrylate was 10.0%. Also, the selectivity of propylene glycol diacrylate was 0.07%.

(Comparative Example 6)

Here, a reaction and a manipulation were conducted in the same manner as Example 11 except that a commercially available ion-exchange resin including a functional group having a structure expressed by General Formula (e) above with a paired anion of $OH^-$ was used as the comparative activating catalyst ②. The ESPD charge of the ion-exchange resin was +1.10 (electrons).

Consequently, the conversion ratio of acrylic acid was 77.4%, and the selectivity of hydroxy ethyl acrylate was 82.5%, while the selectivity of diethylene glycol monoacrylate was 6.2%. Also, the selectivity of ethylene glycol diacrylate was 0.40%. Thus, the foregoing results reveal that the conversion ratio of acrylic acid was reduced when the comparative activating catalyst ② was used.

(Comparative Example 7)

Here, a reaction and a manipulation were conducted in the same manner as Example 12 except that the ion-exchange resin used in Comparative Example 6 was used as the comparative activating catalyst ②. Consequently, the conversion ratio of acrylic acid was 53.9%, and the selectivity of hydroxy propyl acrylate was 82.4%, while the selectivity of dipropylene glycol monoacrylate was 15.2%. Also, the selectivity of propylene glycol diacrylate was 0.08%. Thus, the foregoing results reveal that the conversion ratio of acrylic acid was reduced when the comparative activating catalyst ② was used.

(Example 17)

Here, a hydration reaction of ethylene oxide, which is an addition reaction of a cyclic heterocompound to water, was conducted using the polymer ac-$Cl^-$ synthesized in the manner described in Example 13 as the activating catalyst ②.

More specifically, 40 g of ion-exchanged water and 0.7 g of the activating catalyst ② were charged to an autoclave equipped with a thermometer, a gas conduit, a stirrer, etc. Then, the autoclave was sealed airtight, and pressure was applied to the same with a nitrogen gas, after which the internal temperature thereof was raised to 95°C. Then, 4.9 g of ethylene oxide was introduced to the reaction vessel through the gas conduit at 95°C with stirring of the content thereof. Then, the reaction solution was subjected to maturation for 1 hour at 95°C, whereby the hydration reaction of ethylene oxide was completed.

When the reaction ended, the autoclave was cooled. Then, the reaction solution was filtered, and the filtrate was analyzed by means of GC. Consequently, the conversion ratio of ethylene oxide was 75%, and the selectivity of ethylene glycol was 92%, while the selectivity of diethylene glycol was 8%.

It is understood that embodiments and examples in the Most Preferred Embodiment clause to implement the present invention were presented with intention to make techniques of the present invention clear, and the present invention should not be construed limitedly to these embodiments and examples, and may be varied in many ways within the scope thereof.

INDUSTRIAL APPLICABILITY

The activating catalyst of the present invention can efficiently activate the active hydrogen included in a compound by using an atom (including a nitrogen atom forming the cyclic quaternary ammonium group) showing the ESPD charge within a particular range as the active site. In other words, the catalyst is suitable as an activating catalyst in various kinds of reactions involving the activation of the active hydrogen. Also, by using the activating catalyst, various effects can be attained, for example, a reaction with excellent selectivity can be conducted.

Particularly, by using the activating catalyst, there can be attained an effect that unsaturated carboxylic acid

ester is manufactured efficiently from unsaturated carboxylic acid with good selectivity. Further, by using the activating catalyst, there can be attained an effect that (meth)acrylic acid ester is efficiently manufactured from (meth)acrylic acid with good selectivity.

According to the method of activating the active hydrogen of the present invention, the active hydrogen included in a compound can be activated efficiently by using an atom (including a nitrogen atom forming the cyclic quaternary ammonium group) showing the ESPD within the specific range in the activating catalyst as the active site. Thus, various kinds of reactions involving the activation of the active hydrogen can be conducted. In short, the effect of conducting a reaction with excellent selectivity can be attained.

According to the method of evaluating the activating catalyst of the present invention, by computing the ESPD charge of the atom (including a nitrogen atom forming the cyclic quaternary ammonium) forming a functional group included in the catalyst, it is readily evaluated whether or not the catalyst has an active site, at which the active hydrogen included in the compound can be efficiently activated. In short, the effect of readily evaluating the catalytic ability of the catalyst can be attained.

## Claims

**1.** An activating catalyst for an active hydrogen which activates the active hydrogen included in a compound, characterized in that:

an ESPD charge of at least one atom in an atomic group forming a functional group included in said catalyst is in a range between -0.65 and -1.05 (electrons).

**2.** The activating catalyst of Claim 1, wherein the atom showing the ESPD charge in said range is at least one kind of atom selected from a group consisting of a nitrogen atom, an oxygen atom, and a sulfur atom.

**3.** The activating catalyst of Claim 1, wherein the functional group including the atom showing the ESPD charge in said range is a cyclic amino group.

**4.** An activating catalyst for an active hydrogen which activates the active hydrogen included in a compound, characterized in that:

said catalyst has a cyclic quaternary ammonium group as a functional group; and
an ESPD charge of a nitrogen atom forming said ammonium group is in a range between +0.4 and +1.3 (electrons).

**5.** The activating catalyst of Claim 4, wherein said cyclic quaternary ammonium group has 5 or 6 members.

**6.** The activating catalyst of any of the preceding claims, wherein said catalyst is a crosslinking macromolecule.

**7.** The activating catalyst of Claim 6, wherein said crosslinking macromolecule is in the form of gel.

**8.** The activating catalyst of any of the preceding claims, wherein the compound including the active hydrogen has at least one kind of functional group selected from the group consisting of a -OH group, a -NH-group, and a -COOH group.

**9.** The activating catalyst of any of the preceding claims, wherein the compound including the active hydrogen is at least one kind of compound selected from the group consisting of unsaturated carboxylic acid, alcohol, and water.

**10.** A method of activating an active hydrogen, characterized by activating the active hydrogen included in a compound using the activating catalyst of any of claims 1 through 7.

**11.** A method of evaluating a catalytic ability of a catalyst to activate an active hydrogen included in a compound, characterized by evaluating the catalytic ability by an ESPD charge of an atom forming a functional group included in said catalyst.

## INTERNATIONAL SEARCH REPORT

International application No.
PCT/JP99/02438

A. CLASSIFICATION OF SUBJECT MATTER
Int.Cl$^6$ B01J31/06

According to International Patent Classification (IPC) or to both national classification and IPC

B. FIELDS SEARCHED

Minimum documentation searched (classification system followed by classification symbols)
Int.Cl$^6$ B01J21/00-38/74

Documentation searched other than minimum documentation to the extent that such documents are included in the fields searched
Jitsuyo Shinan Koho 1926-1996 Toroku Jitsuyo Shinan Koho 1994-1999
Kokai Jitsuyo Shinan Koho 1971-1999 Jitsuyo Shinan Keisai Koho 1996-1999

Electronic data base consulted during the international search (name of data base and, where practicable, search terms used)
JICST File (JOIS) ESPD, Seiden Potensharu, Denka
WPI/L (QUESTEL) ESPD, ELECTROSTATIC-POTENTIAL, DERIVE

C. DOCUMENTS CONSIDERED TO BE RELEVANT

| Category* | Citation of document, with indication, where appropriate, of the relevant passages | Relevant to claim No. |
|---|---|---|
| X | JP, 9-310062, A (Nippon Shokubai Co., Ltd.), 2 December, 1997 (02. 12. 97), Full text & EP, 841350, A1 | 1-10 |
| X | JP, 9-313924, A (Nippon Shokubai Co., Ltd.), 9 December, 1997 (09. 12. 97), Full text & EP, 841350, A1 | 1-3, 6-10 |
| X | JP, 9-309924, A (Nippon Shokubai Co., Ltd.), 2 December, 1997 (02. 12. 97), Full text & EP, 841350, A1 | 1-3, 6, 8-10 |
| X | JP, 9-309865, A (Nippon Shokubai Co., Ltd.), 2 December, 1997 (02. 12. 97), Full text & EP, 841350, A1 | 1-3, 6, 8-10 |
| A | JP, 59-133372, A (Bayer AG.), 31 July, 1984 (31. 07. 84), Claims & EP, 115006, A1 & DE, 3248778, A1 & US, 4563371, A | 1-11 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 5 August, 1999 (05. 08. 99) | 17 August, 1999 (17. 08. 99) |
| Name and mailing address of the ISA/ Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP99/02438

C (Continuation). DOCUMENTS CONSIDERED TO BE RELEVANT

| Category* | Citation of document, with indication, where appropriate, of the relevant passages | Relevant to claim No. |
|---|---|---|
| A | JP, 49-31631, B1 (Nitto Boseki Co., Ltd.),<br>23 August, 1974 (23. 08. 74),<br>Claims (Family: none) | 1-11 |

Form PCT/ISA/210 (continuation of second sheet) (July 1992)